# EUROPÄISCHE PATENTSCHRIFT

(11) **EP 1 362 096 B1**
(45) Veröffentlichungstag und Bekanntmachung des Hinweises auf die Patenterteilung: **19.08.2009**
(21) Anmeldenummer: 02716797.2
(22) Anmeldetag: 21.02.2002
(51) Int. Cl.: C12N 5/10, A61K 35/44, A61K 48/00, A61P 27/02

(54) **PIGMENTEPITHELZELLE DES AUGES, IHRE HERSTELLUNG UND VERWENDUNG IN DER THERAPIE EINER AUGEN- ODER ZNS-ERKRANKUNG**
PIGMENTARY EPITHELIUM CELL OF THE EYE, THE PRODUCTION THEREOF AND THE USE OF THE SAME FOR TREATING DISEASES OF THE EYE OR OF THE CENTRAL NERVOUS SYSTEM
CELLULE EPITHELIALE PIGMENTAIRE DE L'OEIL, SA PRODUCTION ET SON UTILISATION DANS LE TRAITEMENT D'AFFECTIONS DES YEUX OU DU SYSTEME NERVEUX CENTRAL

(30) Priorität: 21.02.2001 DE 10108412; 22.02.2001 US 270746 P
(43) Veröffentlichungstag der Anmeldung: 19.11.2003
(73) Patentinhaber: CEVEC Pharmaceuticals GmbH, 51105 Köln (DE)
(72) Erfinder: KOCHANEK, Stefan, 50935 Köln (DE); SCHRAERMEYER, Ulrich, 41466 Neuss (DE); THUMANN, Gabriele, 50931 Köln (DE)
(74) Vertreter: Dehmel, Albrecht
(86) Internationale Anmeldenummer: PCT/EP2002/001853
(87) Internationale Veröffentlichungsnummer: WO 2002/066620

(56) Entgegenhaltungen:
- WO-A-00/15822
- WO-A-99/34834
- KOCHANEK S: "DEVELOPMENT OF HIGH-CAPACITY ADENOVIRAL VECTORS FOR GENE THERAPY" THROMBOSIS AND HAEMOSTASIS, STUTTGART, DE, Bd. 82, Nr. 2, August 1999 (1999-08), Seiten 547-551, XP000892384 ISSN: 0340-6245
- SCHIEDNER G ET AL: "GENOMIC DNA TRANSFER WITH A HIGH-CAPACITY ADENOVIRUS VECTOR RESULTS IN IMPROVED IN VIVO GENE EXPRESSION AND DECREASED TOXICITY" NATURE GENETICS, NEW YORK, NY, US, Bd. 18, Februar 1998 (1998-02), Seiten 180-183, XP000872455 ISSN: 1061-4036
- REH T A ET AL: "RETINAL PIGMENTED EPITHELIAL CELLS INDUCED TO TRANSDIFFERENTIATE TO NEURONS BY LAMININ" NATURE (LONDON), Bd. 330, Nr. 6143, 1987, Seiten 68-71, XP002214403 ISSN: 0028-0836
- RIBEAUDEAU F ET AL: "IN VIVO ADENOVIRUS-MEDIATED GENE TRANSFER TO NEWBORN RAT RETINAL PIGMENT EPITHELIAL CELLS. TRANSFERT DE GENES IN VIVO DANS LES CELLULES DE L'EPITHELIUM PIGMENTAIRE DU RAT NOUVEAU-NE AU MOYEN D'UN ADENOVIRUS RECOMBINANT" COMPTES RENDUS DES SEANCES DE L'ACADEMIE DES SCIENCES. SERIE III: SCIENCES DE LA VIE, ELSEVIER, AMSTERDAM, NL, Bd. 320, Nr. 7, Juli 1997 (1997-07), Seiten 523-532, XP004270089 ISSN: 0764-4469
- RAKOCZY PIROSKA E ET AL: "Development of gene therapy-based strategies for the treatment of eye diseases." DRUG DEVELOPMENT RESEARCH, Bd. 46, Nr. 3-4, März 1999 (1999-03), Seiten 277-285, XP008008112 ISSN: 0272-4391
- AKIMOTO M ET AL: "ADENOVIRALLY EXPRESSED BASIC FIBROBLAST GROWTH FACTOR RESCUES PHOTORECEPTOR CELLS IN RCS RATS" INVESTIGATIVE OPHTHALMOLOGY & VISUAL SCIENCE, ASSOCIATION FOR RESEARCH IN VISION AND, US, Bd. 40, Nr. 2, 1999, Seiten 273-279, XP000917329 ISSN: 0146-0404
- KREPPEL FLORIAN ET AL: "Long-term transgene expression in the RPE after gene transfer with a high-capacity adenoviral vector." IOVS, Bd. 43, Nr. 6, Juni 2002 (2002-06), Seiten 1965-1970, XP008008452 June, 2002

## Beschreibung

Die vorliegende Erfindung betrifft eine Pigmentepithelzelle des Auges enthaltend Vektor-DNA eines adenoviralen Vektors mit großer DNA-Kapazität, die verbessert Isolierung und Kultivierung dieser Zellen und Verfahren zur Herstellung sowie die Verwendung in der Therapie einer Augen- oder Nervenerkrankung.

Die fünf Primärsinne Tasten, Sehen, Hören, Schmecken und Riechen dienen der Aufnahme von Information aus der Umwelt. Ca. 75 % unserer Wahrnehmungen laufen über den Gesichtssinn. Dieser hohe Prozentsatz verdeutlicht die herausragende Bedeutung des Sehens in unserem täglichen Leben. Folglich stellt eine Schwächung unserer Sehkraft einen immensen Einschnitt in den Lebensalltag dar.

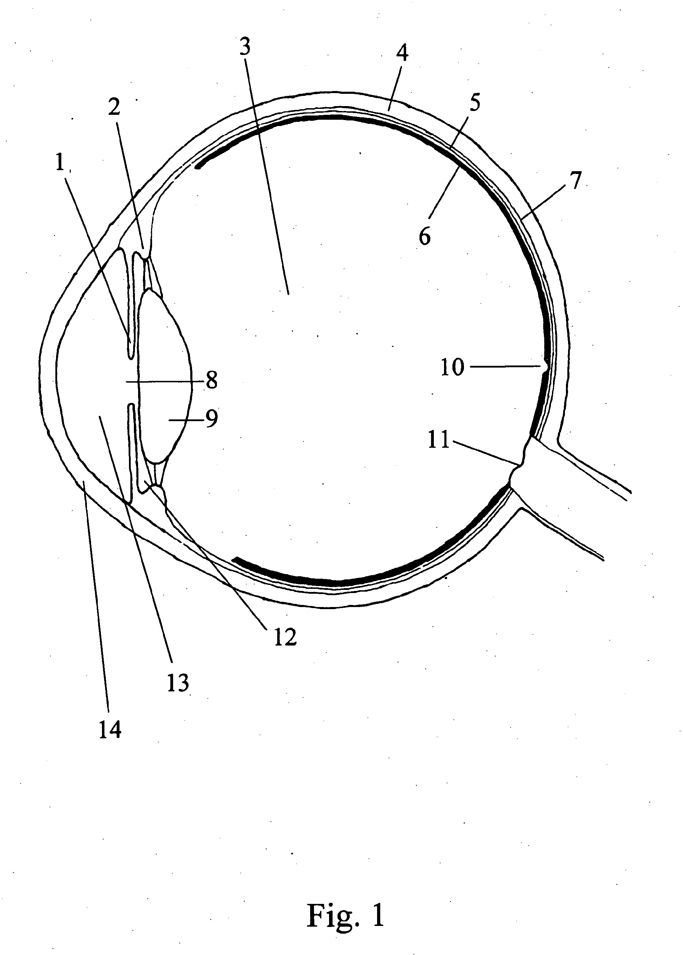

Das Auge besteht aus einem zusammengesetzten Linsensystem, das auf der Netzhaut ein umgekehrtes und verkleinertes Bild der Umwelt darstellt. Der dioptische Apparat besteht aus der durchsichtigen Kornea (Hornhaut), der die Pupille bildenden Iris, der Linse und dem Glaskörper, einer gallertartigen, durchsichtigen Masse im Inneren des Augapfels zwischen Linse und Retina. Fig. 1 zeigt einen schematisierten Horizontalschnitt durch das Auge. Die Hülle des Augapfels besteht aus 3 Schichten: der Sklera (Lederhaut), der Choroidea (Aderhaut) und der Retina (Netzhaut). Die Retina wiederum besteht aus einer äußeren Schicht, dem retinalen Pigmentepithel (RPE), sowie einer inneren Schicht, der neurosensorischen Netzhaut.

Die ringförmige Regenbogenhaut (Iris) trennt die vordere von der hinteren Augenkammer und bildet den vorderen Teil der Uvea (Gefäßhaut). Sie besteht von anterior nach posterior aus einer kollagenreichen Extrazellularmatrix, dem Irisstroma (es enthält Melanozyten, Fibrozyten, Nerven und Blutgefäße) und dem Irispigmentepithel (IPE).

Das IPE ist zweilagig und besteht aus einer anterioren und einer posterioren Pigmentepithelzellschicht (Freddo TF (1996) Ultrastructure of the iris. Microsc Res Tech 33: 369-389): Die Zellen des posterioren Irispigmentepithels sind durch Tight-Junctions verbunden. Das anteriore Pigmentepithel besitzt zusätzlich glatte Muskelzellen (außer im Bereich des Sphinkters), die zur Dilatation der Iris beitragen (Freddo 1996, supra). Das Irispigmentepithel hat dieselbe embryologische Herkunft wie das retinale Pigmentepithel. Aus einer menschlichen Iris können ca. 2,3 x 10⁵ IPE Zellen gewonnen werden, von denen 90 % in Zellkultur überleben (Hu DN, Ritch R, McCormick SA, Pelton-Henrion K (1992) Isolation and cultivation of human iris pigment epithelium. Invest Ophtalmol Vis Sci 33: 2443-2453). IPE Zellen sind stark pigmentiert und enthalten viel Eumelanin. Melanin hat folgende schützenden Funktionen. Es kann zweiwertige Eisen Ionen (Fe²⁺) und andere toxische Substanzen (z.B. Ca²⁺) binden und damit dem Zellzytoplasma entziehen (Hill HZ (1992) The function of melanin or six blind people examine an elephant. Bioessays 14: 49-56). Melanin kann außerdem durch Redoxreaktionen Fe²⁺ in weniger toxisches Fe³⁺ überführen. Andererseits haben die Vorstufen der Melaninsynthese Dihydroxyindol (DHI) und Dihydroxyindolcarboxylsäure (DHIA) eine sehr starke antioxidative Wirkung, die stärker ist als die von alpha Tocopherol (Memoli S, Napolitano A, d'Ischia M, Misuraca G, Palumbo A, Prota G (1997) Diffusible melanin-related metabolites are potent inhibitors of lipid peroxidation. Biochim Biophys Acta 1346: 61-68). Melanin kann toxische Sauerstoffradikale, die im Auge durch den hohen Sauerstoffpartialdruck in Kombination mit Lichtexposition entstehen, eliminieren. Auch Elemente, die für die normale Funktion der Retina wichtig sind, wie beispielsweise Zink, werden mit hohem Wirkungsgrad vom Melanin gespeichert. Zink hat als Kofaktor für zum Beispiel antioxidative Enzyme (Superoxid-dismutase) oder bindegewebsabbauende Enzyme (Metalloproteinasen) mehrere wichtige Bedeutungen im Auge und im zentralen Nervensystem (ZNS).

Das Pigmentepithel spielt eine wichtige Rolle im Stoffwechsel und bei der Lichtabsorption des Auges. Es ist außerdem verantwortlich für die äußere Blut-Retina-Schranke sowie für die Entsorgung abgestoßener Photorezeptorzellen. Folglich bildet sie ein interessantes Ziel zur gentherapeutischen Behandlung von Augenerkrankungen.

Bisher wurden einige Experimente zur genetischen Modifikation von Pigmentepithelzellen beschrieben, die jedoch im Hinblick auf Dauer und Stabilität der Expression keine zufriedenstellenden Ergebnisse lieferten.

In einer Studie mit Labormäusen wurde ein adenoviraler Vektor der ersten Generation für den Gentransfer in das retinale Pigmentepithel durch subretinale Injektion verwendet, der das E.coli lacZ Gen unter Kontrolle des CMV Promotors exprimierte. Adenovirale Vektoren der ersten Generation (Gilardi et al., FEBS Letters 267, 60-62, 1990; Stratford-Perricaudet et al., Hum. Gene Ther. 1, 241-256, 1990) sind durch Deletionen der E1A- und E1B-Gene charakterisiert. E1A und E1B haben transformierende und transaktivierende Eigenschaften. In einigen Vektoren ist außerdem E3 deletiert, um die Kapazität für die Aufnahme fremder DNA zu erhöhen. Obwohl der Gentransfer in das retinale Pigmentepithel effizient war und kurz nach Injektion eine sehr gute Expression im retinalen Pigmentepithel beobachtet wurde, war die Expression transient. 6 Wochen nach Injektion waren nur noch vereinzelt lacZ positive retinale Pigmentepithelzellen zu beobachten (Li T, Adamian M, Roof DJ, Berson EL, Dryja TP, Roessler BJ, Davidson BL (1994) In vivo transfer of a reporter gene to the retina mediated by an adenoviral vector. Invest Ophthalmol Vis Sci: 35, 2543-2549).

In einer weiteren Untersuchung, die an Laborratten mit einem Beobachtungszeitraum von 14 Tagen durchgeführt wurde, wurde ein Adenovirus der ersten Generation verwendet, der das E.coli lacZ Gen unter Kontrolle des Rous Sarcoma Virus (RSV) Promotors exprimierte. Obwohl der Gentransfer in das retinale Pigmentepithel effizient war und 7 Tage nach Injektion eine sehr gute Expression im retinalen Pigmentepithel beobachtet wurde, war die Expression eine Woche später reduziert (Rakoczy PE, Lai CM, Shen WY, Daw N, Constable IJ (1998) Recombinant adenovirus-mediated gene delivery into the rat retinal pigment epithelium in vivo. Australian and New Zealand Journal of Ophthalmology 26 (Suppl.): S56-S58).

In einer anderen Studie, die an 6 Wochen alten RCS Ratten durchgeführt wurde, wurde ein adenoviraler Vektor der ersten Generation verwendet, der das green fluorescent protein (GFP) Gen unter Kontrolle des CMV Promotors exprimierte (Anglade E, Csaky KG (1998) Recombinant adenovirus-mediated gene transfer into the adult rat retina. Curr Eye Res 17: 316-321). Obwohl der Gentransfer in das retinale Pigmentepithel nach subretinaler Injektion effizient war und 3 Tage nach Injektion 30 bis 90 % des retinalen Pigmentepithels im Bereich der Injektionsstelle GFP positiv waren, war 6 Tage später (also 9 Tage nach Injektion) keine GFP Expression mehr nachweisbar.

Während bei den oben genannten Beispielen des Gentransfers im Bereich des Auges adenovirale Vektoren der ersten Generation verwendet wurden, wurde in einer weiteren Publikation für die subretinale Injektion von Mäusen ein adenoviraler Vektor verwendet, der als Adenovirus Minichromosom (EAM) bezeichnet wird (Kumar-Singh R, Farber DB (1998) Encapsidated adenovirus mini-chrorilosome-mediated delivery of genes to the retina: application to the rescue of photoreceptor degeneration. Hum Mol Genet 7: 1893-1900). Hierbei handelt es sich um einen Vektor, der keine Virusproteine exprimiert. Der Vektor exprimierte die beta Einheit der zyklischen GMP Phosphodiesterase (PDE) unter der Kontrolle des natürlichen PDE Promotors. Ferner exprimierte der Vektor das E.coli lacZ Gen unter Kontrolle des CMV Promotors. Außerdem enthielt der Vektor verschiedene E.coli Plasmidelemente (Plasmid backbone, Ampicillin Resistenzgen, E. coli Ursprung der Replikation). Nach Produktion war der Vektor durch ausgeprägte Variabilität seines Genoms charakterisiert. Monomere und dimere Strukturen wurden beobachtet, letztere in Kopf-Kopf, Kopf-Schwanz und Schwanz-Schwanz Orientierung (head-to-head, head-to-tail und tail-to-tail). Wegen dieser Variabilität und des Vorliegens von Plasmidsequenzen inklusive Antibiotikaresistenz ist dieser Vektor nicht für den therapeutischen Einsatz geeignet. Die Gentransferexperimente wurden an *rd* Mäusen durchgeführt, die ein Tiermodell für retinale Degeneration darstellen und durch eine für die Degeneration ursächliche Mutation in der beta Einheit des PDE Gens charakterisiert sind. In dieser Studie wurde die Expression ausschließlich im neuronalen Anteil der Retina nachgewiesen, nicht jedoch im retinalen Pigmentepithel. Obwohl die neuronalen Zellen post-mitotisch sind und sich also nicht mehr teilen können, war die Expression des PDE Gens nur transient. Durch verschiedene Methoden (RT-PCR, Western Blot Analyse und Bestimmung der PDE Aktivität) wurde nachgewiesen, daß 4 Monate nach Injektion keine Expression mehr nachweisbar war.

Für die Transfektion von IPE Zellen ist bisher lediglich das nicht-virale Transfektionsreagenz Lipofectamine verwendet worden. In dieser Untersuchung wurde das Plasmid pXCN2-bFGF hergestellt, das die bFGF cDNA der Ratte exprimiert. Zusätzlich enthält das Plasmid eine Neomycin Resistenz Gen. Kultivierte IPE Zellen der Ratte wurden mit diesem Plasmid transfiziert. Die Zellen exprimierten die bFGF cDNA in vitro und die Autoren schreiben, daß nach subretinaler Transplantation als Zellsuspension in der RCS Ratte die Degeneration von Photorezeptoren bis zu 4 Wochen verzögert wurde (Tamai M, Yamada K, Takeda N, Tomita H, Abe T, Kojima S, Ishiguro I (1997) bFGF transfected iris pigment epithelial cells rescue photoreceptor cell degeneration in RCS rats. In: La Vail M, eds. Degenerative retinal diseases. 323-328). Da jedoch, wie in der genannten Arbeit gezeigt, auch in Ratten, die IPE Zellen nach Transfektion mit einem Kontrollplasmid durch subretinale Injektion erhalten hatten, der gleiche Effekt, nämlich Verzögerung der Degeneration von Photorezeptoren beobachtet wurde, der durch die Transfektion nicht verbessert oder verlängert wurde, handelte es sich hier nicht um einen Effekt, der auf einen gezielten Gentransfer zurückgeführt werden konnte, sondern der allein durch die Transplantation der IPE zellen, zu erklären war. Außerdem wurde kein Nachweis einer bFGF Expression nach der Transplantation geführt.

In WO0015822 ist die Verwendung isolierter DNA-Segmente, rekombinanter Vektoren und rekombinanter Wirtszellen beschrieben, die Wirkstoffe kodieren bzw. exprimieren, welche zur Inhibierung der Degradation von Photorezeptorzellen nützlich sind. Für den nötigen Gentransfer in retinale Pigemtepithelzellen wird dabei die Verwendung von adenoassoziierten Viren beschrieben, wobei das Transgen in das Genom der Wirtszelle integriert ist.

Ein Vergleich von AAV und adenoviralen Vektoren mit großer DNA-Kapazität hinsichtlich ihrer DNA Kapazität und ihrer Herstellung ist in Kochanek, 1999 dargestellt (Kochanek S (1999) Development of high-capacity adenoviral vectors for gene therapy. Thrombosis and jaemostasis, Stuttgart, Bd. 82 Nr. 2: 547-551). Entsprechende Gentransferexperimente wurden jedoch nur in laborstandardisierten, immortalisierten Zelllinien wie 293, KB, NMuLi oder BNL CL.2 durchgeführt.

Die genannten Publikationen offenbaren daher kein Expressionssystem für Pigmentepithelzellen des Auges, mit dem eine langfristige und stabile Expression eines eingeschleusten Gens beobachtet werden kann. Eine langfristige und stabile Expression eines solchen Gens ist zur Therapie einer Vielzahl von angeborenen und erworbenen Erkrankungen des Auges aber erforderlich. Für viele Anwendungen ist gerade die langfristige Produktion von therapeutischen Proteinen der entscheidende Faktor für das Erzielen eines therapeutischen Effekts.

Aufgabe der vorliegenden Erfindung ist es daher, Pigmentepithelzellen des Auges zur Verfügung zu stellen, die therapeutisch einsetzbar sind.

Die Aufgabe wird gelöst durch die Bereitstellung einer erfindungsgemäßen Pigmentepithelzelle des Auges.

Überraschenderweise wurde nun gefunden, daß eine Pigmentepithelzelle des Auges, die Vektor-DNA eines adenoviralen Vektors mit großer DNA-Kapazität enthält, langfristig stabil mindestens ein eingeführtes Gen exprimiert und somit therapeutisch einsetzbar ist.

Ein Gegenstand der vorliegenden Erfindung ist eine Pigmentepithelzelle des Auges, die eine Vektor-DNA eines adenoviralen Vektors mit großer DNA-Kapazität enthält.

Unter einer Pigmentepithelzelle des Auges wird eine Epithelzelle des Auges verstanden, in der Pigment, beispielsweise Melanin, eingelagert ist. Ein Beispiel für eine Pigmentepithelzelle des Auges ist eine retinale Pigmentepithelzelle (RPE) oder eine Irispigmentepithelzelle (IPE).

Unter einem adenoviralen Vektor großer DNA-Kapazität versteht der Fachmann Adenoviren, die keine viralen kodierenden DNA-Sequenzen enthalten (Kochanek S, Clemens PR, Mitani K, Chen HH, Chan S, Caskey CT (1996) A new adenoviral vector: Replacement of all viral coding sequences with 28 kb of DNA independently expressing both full-length dystrophin and beta-galactosidase. Proc Natl Acad Sci U.S.A. 93: 5731-5736; Fisher KJ, Choi H, Burda J, Chen SJ, Wilson JM. (1996); Recombinant adenovirus deleted of all viral genes for gene therapy of cystic fibrosis. Virology 217: 11-22; Kumar-Singh R, Chamberlain JS (1996) Encapsidated adenovirus minichromosomes allow delivery and expression of a 14 kb dystrophin cDNA to muscle cells. Hum Mol Genet 5: 913-921). Diese Adenoviren enthalten lediglich die viralen Enden unter Einschluß der "inverted terminal repeats" (ITRs) und des Verpackungssignals. Die Kapazität für die Aufnahme fremder DNA beträgt beispielsweise bis zu etwa 37 kb, da der weit überwiegende Teil des adenoviralen Genoms entfernt ist.

Adenoviren sind als Expressionsvektoren, insbesondere im Rahmen der Gentherapie, von besonderer Bedeutung. Ein Vorteil von adenoviralen Vektoren ist die Tatsache, daß diese Vektoren replizierende und nicht-replizierende Zellen *in vitro* und *in vivo* effizient transduzieren können.

Es sind verschiedene Systeme zur Herstellung adenoviraler Vektoren großer DNA-Kapazität beschrieben worden (Kochanek S (1999) High-capacity adenoviral vectors for gene transfer and somatic gene therapy. Hum Gene Ther 10: 2451-2459). Der Vorteil dieser adenoviralen Vektoren mit großer DNA-Kapazität im Vergleich zu adenoviralen Vektoren der ersten und zweiten Generation liegt vor allem in der größeren Kapazität. Hierdurch ist es möglich, ein oder mehrere Gene oder Expressionskassetten in die Pigmentepithelzellen einzuführen.

Nach Aufnahme des adenoviralen Vektors in die Zelle wird die Hülle des Vektor üblicherweise in Endosomen abgebaut. Die verbleibende Vektor-DNA wird dann in den Zellkern transportiert und integriert, in der Regel jedoch nicht in das zelluläre Genom.

Ein Beispiel für einen adenoviralen Vektor mit großer DNA-Kapazität ist ein Vektor, der das menschliche alphal-Antitrypsin Gen exprimiert (Schiedner G, Morral N, Parks RJ, Wu Y, Koopmans SC, Langston C, Graham FL, Beaudet AL, Kochanek S, (1998) Genomic DNA transfer with a high capacity adenovirus vector results in improved in vivo gene expression and decreased toxicity. Nature Genetics 18: 180-183). Ein weiteres Beispiel ist eine Vektor, der das Dystrophin Gen und das E.coli lacZ Gen exprimiert (Kochanek S, Clemens PR, Mitani K, Chen HH, Chan S, Caskey CT (1996) A new adenoviral vector: Replacement of all viral coding sequences with 28 kb of DNA independently expressing both full-length dystrophin and beta-galactosidase. Proc Natl Acad Sci U.S.A. 93: 5731-5736) In einer bevorzugten Ausführungsform wird HC-AdFK7 oder HC-AdhCMV.PEDF als adenoviraler Vektor mit großer DNA-Kapazität verwendet. HC-AdFK7 ist ein adenoviraler Vektor mit großer DNA-Kapazität, der das "enhanced green flurorescent" Protein (EGFP) unter der Kontrolle des humanen Zytomegalievirus Promotors exprimiert. HG-AdhCMV.PEDF ist ein adenoviraler Vektors mit großer DNA-Kapazität, der das humane "pigment epithelial cell derived factor" (PEDF) Gen unter der Kontrolle des Zytomegalievirus Promotors exprimiert. In diesem Vektor ist das PEDF Protein durch das Anheften (Expression als Fusionsprotein) eines Poly-Histidin Epitops markiert, so daß das Protein durch Verwendung eines Anti-Poly-Histidin Antikörpers leicht nachgewiesen werden kann.

Wie in den Beispielen gezeigt, sind Pigmentepithelzellen mit einen adenoviralen Vektor großer DNA-Kapazität in vitro sehr effizient transduzierbar. Wie ebenfalls in den Beispielen gezeigt, kommt es nach einer Transplantation dieser genetisch modifizierten Zellen zu einer sehr langfristigen Genexpression, die konstant über mindestens 4 Monate nachweisbar ist. Hierbei ist der Ort der Transplantation offenbar nicht kritisch. Eine Transplantation der genetisch modifizierten Pigmentepithelzellen sowohl in das Auge in den subretinalen Raum als auch besonders überraschend in das ZNS in das Corpus striatum führte zu einer langfristigen über mindestens 4 Monate (Auge) bzw. mindestens 2 Monate (ZNS) nachweisbaren Genexpression.

Die meisten Experimente mit diesen Vektoren wurden bisher in der Leber und in der Skelettmuskulatur durchgerührt. Nach Lebergentransfer mit einem das menschliche alphal-Antitrypsin exprimierenden Vektor in Pavianen (Morral N, O'Neal W, Rice K, Leland M, Kaplan J, Piedra PA, Zhou H, Parks RJ, Velji R, Aguilar-Cordova E, Wadsworth S, Graham FL, Kochanek S, Carey KD, Beaudet AL (1999) Administration of helper-dependent adenoviral vectors and sequential delivery of different vector serotype for long-term liver-directed gene transfer in baboons. Proc Natl Acad Sci USA 96: 12816-12821) kam es in zwei von drei Tieren zwar zu einer längerfristigen Expression (länger als ein Jahr), dennoch wurde im Gegensatz zu der vorliegenden Erfindung eine kontinuierliche Abnahme der Expression beobachtet, die in einem der Tiere nach 16 Monaten noch 19 % und in dem zweiten Tier nach 24 Monaten noch 8 % der Ausgangswerte betrug. In einem dritten Tier kam es zu einem vollständigen Verlust der Expression innerhalb von 10 Wochen. Über den Grund für die langsame Abnahme der Expression in den zwei Tieren mit der verlängerten Expression wurde spekuliert. Sowohl Wachstum der Tiere als auch langsame Zellteilung der Hepatozyten wurden diskutiert. Letztlich ist die Ursache für den langsamen Verlust der Expression nicht geklärt. In dem Tier, in dem ein rascher Verlust der Expression beobachtet wurde, wurde die Entstehung von Antikörpern beobachtet, die gegen das menschliche alphal-Antitrypsin gerichtet waren.

Eine deutlichen Abnahme der Expression eines LacZ Reportergens wurde nach dem Gentransfer in die Leber mit einem adenoviralen Vektor, großer DNA-Kapazität innerhalb von 30 Tagen nach Injektion in einer weiteren Studie beobachtet (Parks RJ, Bramson JL, Wan Y, Addison CL, Graham FL (1999) Effects of stuffer DNA on transgene expression from helper-dependent adenovirus vectors. J Virol 73: 8027-8034).

Die Skelettmuskulatur ist ein Gewebe, das in bezug auf den natürlichen Umsatz [Turnover] der Zellen den Pigmentepithelzellen des Auges ähnelt. Die Skelettmuskelzellen sind postmitotische Zellen. Dies bedeutet, daß sie sich, ähnlich wie Pigmentepithelzellen, nicht mehr teilen. Besondere Erfahrung besteht im Gentransfer in die Skelettmuskulatur von Labortieren unter Verwendung von adenoviralen Vektoren großer DNA-Kapazität. In diesen Experimenten erfolgte der Gentransfer ähnlich wie bei der vorliegenden Erfindung durch direkte Injektion in das Gewebe. Obwohl nach Gentransfer unter Verwendung eines adenoviralen Vektors großer DNA-Kapazität, der sowohl die Dystrophin cDNA als auch das E.coli LacZ Gen exprimierte, eine Expression über einen längeren Zeitraum zu verzeichnen war, wurde dennoch beobachtet, daß die Expression innerhalb von 84 Tagen wiederum ,abnahm (Chen HH, Mack LM, Kelly R, Ontell M, Kochanek S, Clemens PR (1997) Persistence in muscle of an adenoviral vector that lacks all viral genes. Proc Natl Acad Sci USA 94: 1645-1650). In immunokompetenten Tieren, die keine Toleranz gegenüber der E.coli beta-Galaktosidase aufwiesen, kam es zu einem vollständigen Verlust der Expression nach 84 Tagen.

Im Gegensatz zu den in der Literatur beschriebenen Experimenten war die Stabilität der Expression der Gene, die mittels Gentransfer mit einem adenoviralen Vektor großer DNA-Kapazität in die Pigmentepithelzelle eingeschleust worden waren, überraschenderweise groß. Die Vorteile der adenoviralen Vektoren großer DNA-Kapazität bei der erfindungsgemäßen Transfektion von Pigmentepithelzellen des Auges gegenüber den bekannten Transfektionssystemen sind folglich
- die Gewährleistung einer stabilen Genexpression;
- die Möglichkeit, regulierte Genexpression durch Verwendung konstitutiver, gewebespezifischer, regulierbarer Promotoren und regulierbarer Expressionssysteme zu erzielen;
- fehlende Immunogenität und Toxizität des Vektors;
- hohe Transduktionseffizienz bei Verwendung von Pigmentepithelzellen.

In einer bevorzugten Ausführungsform enthält daher der adenovirale Vektor eine therapeutische Nukleinsäure, insbesondere eine therapeutische DNA, die nicht von dem adenoviralen Vektor abstammt. Hierbei könnte es sich beispielsweise um ein therapeutisches Gen handeln. Unter einem therapeutischen Gen versteht der Fachmann ein Gen, dessen Expressionsprodukt zur Therapie oder Diagnose einer Krankheit dienen kann.

Unter einer Nukleinsäure versteht man ein Polymer, das bei Hydrolyse in Zucker, insbesondere Pentosen, vor allem Ribose und Desoxyribose, heterozyklische organische Basen, insbesondere Adenin, Cytosin, Guanin, Thymin und Uracil, und Phosphorsäure gespalten wird. Die Nukleinsäure kann beispielsweise eine DNA oder RNA sein. Eine therapeutische Nukleinsäure ist eine Nukleinsäure, die selbst oder deren Produkt eine therapeutische Wirkung hervorruft.

Unter einem Gen versteht man einen linearen DNA-Abschnitt, der für ein Protein oder eine RNA codiert. Das therapeutische Gen, das durch Gentransfer in die Pigmentepithelzelle eingebracht wird, kann unterschiedlicher Natur sein. Die Wahl wird vom therapeutischen Ziel bestimmt. Zum Beispiel kann ein Gen verwendet werden, das für einen neurotrophen Faktor kodiert. Beispiele für neurotrophe Faktoren sind der "glial cell derived neurotrophic factor" (GDNF) und der "pigment epithelial cell derived factor" (PEDF). Zum Beispiel können auch Gene verwendet werden, die Neoangiogenese verhindern. Ein Beispiel ist der lösliche Rezeptor für den "vascular endothelial cell growth factor" (VEGF) mit der Bezeichnung löslicher "vascular endothelial cell growth factor receptor-1 (sflt1) (Roeckl W, Hecht D, Sztajer H, Waltenberger J, Yayon A, Weich HA (1998) Differential binding characteristics and cellular inhibition by soluble VEFG receptors 1 and 2. Experimental cell research 241: 161-170). Ein weiteres Beispiel ist ein dominant-negativer VEGF Rezeptor 2 (KDR) (Machein MR, Risau W, Plate KH (1999) Antiangiogenic gene therapy in a rat glioma model using a dominant-negative vascular endothelial growth factor receptor 2. Hum Gene Ther 10: 1117-1128). Weitere therapeutische Gene könnten beispielsweise NGF, BDNF, CNTF, bFGF oder Neurotrophin 3,4-5 sein.

PEDF hat sehr starke neurotrophe und neuroprotektive Wirkung (King GL, Suzuma K(2000) Pigment-epithelium-derived factor--a key coordinator of retinal neuronal and vascular functions. N Engl J Med 342: 349-351). Dieser Faktor wird unter normoxischen Bedingungen vom RPE gebildet. Bei Hypoxie wird die Produktion eingestellt. Hierdurch wird die Neovaskularisation stark gefördert. Bei der altersabhängigen Makuladegeneration (AMD) bilden die geschädigten RPE Zellen zu wenig PEDF. Hierdurch wird eine unkontrollierte Gefäßneubildung induziert. Im Auge besteht die zentrale Wirkung von PEDF darin, die Neubildung von Gefäßen zu verhindern.

Gemäß der vorliegenden Erfindung kann daher eine genetisch modifizierte Pigmentepithelzelle eine Pigmentepithelzelle sein, die nach genetischer Modifikation mit einem PEDF exprimierenden adenoviralen Vektor großer DNA-Kapazität PEDF sezerniert. Diese Zelle kann dann beispielsweise in den subretinalen Raum nahe der Makula bei Patienten im Anschluß an die chirurgische Entfernung von Neovaskularisationsmembranen transplantiert werden. Die Pigmentepithelzelle kann so einerseits das entfernte retinale Pigmentepithel ersetzen und andererseits das für die Verhinderung der Neovaskularisation wesentliche PEDF produzieren. Hierdurch wird das Visus stabilisiert. PEDF kann Außerdem vor Glutamat vermittelter Neurotoxizität schützen.

Desweiteren können je nach Krankheitsursache verschiedene therapeutische Gene von dem adenoviralen Vektor großer DNA-Kapazität einzeln oder in Kombination exprimiert werden.

In einer weiteren bevorzugten Ausführungsform steht das Gen unter der Kontrolle eines viralen oder nicht-viralen Promotors, der konstitutiv, gewebespezifisch und/oder regulierbar aktiv ist.

Unter einem konstitutiv aktiven Promotor versteht man einen Promotor, der in nahezu allen Geweben und nahezu unabhängig vom physiologischen Zustand der Zelle die Transkription des nachgeschalteten Gens vermittelt. Ein Beispiel für einen konstitutiv aktiven Promotor ist der SV40- oder der Zytomegalie-Virus-Promotor.

Unter einem gewebespezifischen Promotor versteht man einen Promotor, der die Transkription des nachgeschalteten Gens nur in einem bestimmten Gewebe vermittelt. Die Verwendung des gewebespezifischen Promotors erlaubt die gewebespezifische Expression eines Proteins oder einer funktionellen RNA in IPE oder in RPE Zellen. Ein Beispiel für einen solchen gewebespezifischen Promotor ist der Transthyretin Promotor, der eine gute Aktivität in RPE und in IPE Zellen hat.

Unter einem regulierbaren Promotor versteht man einen Promotor, der beispielsweise in Abhängigkeit von der Stoffwechselsituation der Zelle, der Konzentration eines Moleküls oder der Temperatur die Transkription eines Gens vermittelt. Die Genexpression kann quantitativ und qualitativ durch die Verwendung eines regulierbaren Promotors kontrolliert werden. Ein Beispiel für einen regulierbaren Promotors ist ein Promotor, der durch Einschluss eines Hypoxie-sensitiven Elements im Falle einer Hypoxie aktiviert wird (Boast K, Binley K, Iqball S, Price T, Spearman H, Kingsman S, Kingsman A, Naylor S (1999) Characterization of physiologically regulated vectors for the treatment of ischemic disease. Hum Gene Ther 10: 2197-2208).

Es kann aber auch ein regulierbares Expressionssystem verwendet werden, das beispielsweise bei Gabe eines Medikamentes induziert oder inaktiviert wird. Ein Beispiel für ein solches System ist ein Tetrazyklin-abhängiges Genexpressionssystem (Freundlieb S, Schirra-Muller C, Bujard H (1999) A tetracycline controlled activation/repression system with increased potential for gene transfer into mammalian cells. J Gene Med 1:4-12).

Nach der Transduktion der Pigmentepithelzelle mit einem adenoviralen Vektor mit großer DNA Kapazität kann die Zelle therapeutische Proteine oder RNAs produzieren. Ein therapeutisches Protein ist ein Protein, das eine therapeutische Wirkung hervorruft. Analoges gilt für eine therapeutische RNA, beispielsweise eine anti-sense RNA oder ein Ribozym. Beispiele für therapeutische Proteine sind die neurotrophen Faktoren PEDF, GDNF, NGF, BDNF, CNTF, bFGF oder Neurotrophin 3,4-5 (Friedman WJ, Black IB, Kaplan DR (1998) Distribution of the neurotrophins brain-derived neurotrophic factor, neurotrophin-3, and neurotrophin-4/5 in the postnatal rat brain: an immunocytochemical study Neuroscience 84:101-114) sowie anti-angiogenetisch wirksamen Faktoren, wie zum Beispiel der lösliche VEGF Rezeptor-1 (sflt-1), der dominant-negativen VEGFR-2 (KDR), sowie wiederum PEDF, das neben seiner neurotrophen Funktion auch eine anti-angiogenetische Wirkung hat (Dawson DW, Volpert OV, Gillis P, Crawford SE, Xu H, Benedict W, Bouck NP (1999) Pigment epithelium-derived factor: a potent inhibitor of angiogenesis. Science 285: 245-248).

Weitere Beispiele für therapeutische Gene sind lysosomale Enzyme (Cingle KA, Kalski RS, Bruner WE, 'O'Brien CM, Erhard P, Wyszynski RE (1996) Age-related changes of glycosidases in human retinal pigment epithelium. Curr Eye Res 115: 433-438) alpha-Mannosidase, beta-galactosidase, N-acetyl-beta-glucosaminidase und N-acetyl-beta-galactosaminidase sowie Lipase. Diese Enzyme spielen beim Abbau von Sehzellmembranen eine wichtige Rolle und können bei der AMD vermindert sein.

Noch weitere Beispiele sind Gene, die für antioxidative Enzyme (Superoxiddismutase, Katalase, Peroxydasen) kodieren, da diese ebenfalls in der Pathogenese von AMD eine Rolle spielen können. (Frank RN, Amin RH, Puklin JE (1999) Antioxidant enzymes in the macular retinal pigment epithelium of eyes with neovascular age-related macular degeneration, J Ophthalmol 127:694-709).

Weitere Beispiele sind Gene für Genprodukte, die die Äderhautdurchblutung steigern können, beispielsweise NO-Synthasen; da eine verminderte Aderhautdurchblutung bei der Pathogenese von AMD eine Rolle spielen kann. (Luksch A, Polak K, Beier C, Polska E, Wolzt M, Dorner GT, Eichler HG, Schmetterer L (2000) Effects of systemic NO synthase inhibition on choroidal and optic nerve head blood flow in healthy subjects. Invest Ophthalmol Vis Sci 41: 3080-3084).

Ein weiterer Gegenstand der vorliegenden Erfindung ist eine erfindungsgemäße Pigmentepithelzelle in einem festen Zellverband, einem sog. Zellsheet. Im Rahmen der experimentellen Therapie der AMD wurden bisher immer nur Einzelzellsuspensionen von autologen IPE Zellen transplantiert. Der Vorteil solcher Zellsheets besteht darin, daß man die Zellen transplantationstechnisch deutlich besser plazieren kann und daß das Wegwandern der Zellen vom Transplantationsort verhindert wird. Pigmentepithelzellen in einem festen Zellverband sind dadurch gekennzeichnet, daß der Zellverband aus mindestens ca. 100, bevorzugt ca. 1000, besonders bevorzugt ca. 10000 Pigmentepithelzellen besteht und diese nicht durch moderate Scherkräfte, insbesondere durch wiederholtes, beispielsweise zehnmaliges, Auf-und Abbewegen in einer Lösung mittels Pipette, voneinander getrennt werden können.

Ein weiterer Gegenstand der vorliegenden Erfindung ist eine Kultivierungssystem enthaltend mindestens eine erfindungsgemäße Pigmentepithelzelle des Auges und einen "feeder layer". Üblicherweise ist die Vermehrung von IPE- und RPE Zellen nach ihrer Isolierung sehr langwierig. Durch das erfindungsgemäße Kultivierungssystem lassen sich große Zahlen von IPE- und RPE Zellen in sehr kurzer Zeit produzieren.

Unter einem "feeder layer" versteht der Fachmann Zellen, die mit anderen Zellen (Zielzellen) kokultiviert werden und das Wachstum der Zielzellen positiv beeinflussen. Unter einer positiven Beeinflussung kann beispielsweise ein schnelleres Wachstum der Zellen oder die Verhinderung von Differenzierung oder Dedifferenzierung verstanden werden. Dies geschieht beispielsweise dadurch, daß von den Zellen des "feeder layer" Moleküle in das Medium abgesondert werden, die dann das Wachstum der Zielzelle positiv beeinflussen.

In der Regel werden inaktivierte Fibroblasten als "feeder layer" zur Kultivierung von embryonalen Stammzellen verwendet werden. Die Inaktivierung der Fibroblasten kann beispielsweise durch eine Behandlung mit Mitomycin C oder durch eine γ-Strahlenexposition erreicht werden. Es können beispielsweise Fibroblasten eines Säugetiers, insbesondere der Maus oder des Menschen, verwendet werden. In einer Ausführungsform werden die Fibroblasten und Pigmentepithelzellen der gleichen Spezies, insbesondere des selben Individuums, verwendet. Bei den Fibroblasten kann es sich aber auch um eine permanente Zellinie, beispielsweise STO-Fibroblasten oder 3T3 Fibroblasten, oder um primäre embryonale Fibroblasten handeln. Die Herstellung der Fibroblasten ist dem Fachmann bekannt (z. B. Abbondanzo S, Gadi I, Stewart C (1993) Derivation of embryonic stern cell lines. Methods in Enzymology 225: 803-823).

Das Kultivierungssystem könnte beispielsweise ein Kulturgefäß beinhalten, in dem die Pigmentepithelzellen des Auges und der "feeder layer" in einem geeigneten Medium unmittelbar aneinander angrenzend, insbesondere übereinander, kultiviert werden. Die unterschiedlichen Zellen können aber auch in einem Kulturgefäß räumlich voneinander getrennt kultiviert werden, so daß der Austausch von beispielsweise Faktoren zur Wachstumsstimulation lediglich über das Medium erfolgt.

Ein weiterer Gegenstand der vorliegenden Erfindung ist ein Verfahren zur Herstellung einer Pigmentepithelzelle des Auges, wobei die Zelle mit Hilfe eines adenoviralen Vektors großer DNA-Kapazität genetisch modifiziert wird.

Unter einer genetischen Modifikation versteht der Fachmann jegliche Verän-derung der Erbinformation der Zellen. Dies kann beispielsweise durch ein- oder mehrfache Nukleotidaddition, -insertion, -substitution und/oder -deletion erreicht werden. In einer besonderen Ausführungsform wird die genetische Modifikation durch Gentransfer herbeigeführt, wobei das Gen beispielsweise extrachromosomal in der Zelle vorliegen kann.

Unter Gentransfer versteht man das Einbringen eines oder mehrerer Gene in beispielsweise eine Zelle. In der vorliegenden Erfindung kann beispielsweise mindestens ein Gen mit Hilfe eines adenoviralen Vektors mit großer DNA-Kapazität in eine Pigmentepithelzelle eingebracht werden. Üblicherweise werden cDNAs verwendet. Es können jedoch auch die Gene selbst (einschließlich ihrer Introns und Exöns) verwendet werden. In einer anderen Ausführungsform können aber auch ein genetisch modifiziertes, natürlich vorkommendes Gen oder künstliche Nukleinsäuren in die Pigmentepithelzelle eingeführt werden.

Ein weiterer Gegenstand der vorliegenden Erfindung ist ein Verfahren zur Herstellung einer erfindungsgemäßen Pigmentepithelzelle des Auges enthaltend einen adenoviralen Vektor großer DNA-Kapazität, wobei die Zelle in Serum-freiem Medium, in Gegenwart eines "feeder layer" und/oder in einem festen Zellverband kultiviert wird.

Zur Isolierung von Pigmentepithelzellen in einem festen Zellverband wird die Iris oder ein Teil der Iris oder die Retina, insbesondere im peripheren retinalen Bereich, beispielsweise mechanisch oder enzymatisch, insbesondere mit Accutase, Chondroitinase und/oder Heparinase, insbesondere vom Stroma und der Basalmembran, getrennt. Das Zellsheet kann dann in Zellkultur weiter kultiviert werden. Bei Bedarf können die Zellsheets durch erneute Accutaseinkubation in Einzelzellen zerlegt werden.

In Rahmen der vorliegenden Erfindung konnte gezeigt werden, daß ein Zellkulturmedium, das kein Serum, beispielsweise foetales Kälberserum, enthält, das Wachstum der Pigmentepithelzellen des Auges positiv beeinflußt.

Ein weiterer Gegenstand der vorliegenden Erfindung ist die Verwendung von erfindungsgemäßen Pigmentepithelzellen zur Therapie von Augenerkrankungen, wobei es sich sowohl um eine angeborene als auch um eine erworbene Augenerkrankung handeln kann. Beispiele für erworbene oder angeborene Augenerkrankungen sind die altersabhängige Makuladegeneration, das Glaukom, und die diabetische Retinopathie.

Die altersabhängige Makuladegeneration (AMD) ist der häufigste Grund für die gesetzliche Erblindung in westlichen Ländern. Durch Atrophie des submakulären retinalen Pigmentepithels und durch die Entwicklung choroidaler Neovaskularisationen (CNV) kommt es sekundär zum Verlust der zentralen Sehschärfe. Für die Mehrzahl der Patienten mit subfovealer CMV und geographischer Atrophie ist derzeit keine Behandlung zur Vermeidung des Verlustes der zentralen Sehschärfe verfügbar. Frühe Zeichen von AMD sind Ablagerungen (Drusen) zwischen retinalem Pigmentepithel und der Bruch's Membran. Im Verlauf der Erkrankung kommt es zur Einsprossung von Aderhautgefäßen in den subretinalen Raum der Makula. Dies führt zum Verlust des zentralen Sehens und der Lesefähigkeit. Ein Beispiel für ein therapeutisches Gen, das für die Therapie der AMD eingesetzt werden kann, ist das PEDF Gen.

Glaukom bezeichnet eine Gruppe von Krankheiten, bei denen der Druck im Auge anomal ansteigt. Dies führt zu Einschränkungen des Gesichtsfeldes und zu einem allgemeinen Nachlassen der Sehfähigkeit. Die häufigste Form ist das primäre Glaukom; hier werden zwei Formen unterschieden: das chronische Weitwinkel-Glaukom und die akute Winkelblockung. Das sekundäre Glaukom kann durch Infektionen, Tumore oder Verletzungen verursacht werden. Ein dritter Typ, das angeborene Glaukom, geht in der Regel auf Entwicklungsstörungen während der Schwangerschaft zurück. Die Kammerflüssigkeit im Augapfel steht unter einem bestimmten Druck, der für die optischen Eigenschaften des Auges notwendig ist. Dieser Augeninnendruck beträgt normalerweise 15 bis 20 Millimeter Quecksilbersäule und wird durch das Gleichgewicht zwischen Flüssigkeitsproduktion und Flüssigkeitsabfluss gesteuert. Beim Glaukom ist der Abfluss der Kammerflüssigkeit im Winkel der vorderen Augenkammer blockiert, so dass der Druck im Augeninneren ansteigt. Meist entwickelt sich das Glaukom im mittleren oder fortgeschrittenen Alter, aber auch angeborene Formen sowie Erkrankungen bei Kindern und Jugendlichen sind nicht selten. Obwohl der Augeninnendruck nur mäßig erhöht ist und ansonsten keine Symptome zu erkennen sind, treten schleichende Schäden auf, insbesondere eine Einschränkung des Gesichtsfeldes. Die akute Winkelblockung dagegen, verursacht Schmerzen, Rötungen, eine Erweiterung der Pupillen und schwere Sehstörungen. Die Hornhaut trübt sich, und der Augeninnendruck ist stark erhöht. Im weiteren Verlauf der Krankheit wird das Gesichtsfeld immer enger, was sich mit dem Perimeter, einem augenärztlichen Instrument, leicht nachweisen lässt. Das chronische Glaukom spricht im Allgemeinen gut auf lokal verabreichte Medikamente an, die den Flüssigkeitsabfluss verstärken. Zur Verminderung der Flüssigkeitsproduktion werden manchmal systemische Wirkstoffe gegeben. Nicht immer jedoch ist die medikamentöse Behandlung erfolgreich. Bei Versagen der medikamentösen Therapie kommen Lasertherapie oder herkömmliche Operationen zum Einsatz, um einen neuen Abfluss für die Kammerflüssigkeit zu schaffen. Das akute Glaukom ist ein medizinischer Notfall. Wird der Augeninnendruck nicht innerhalb von 24 Stunden gesenkt, treten dauerhafte Schäden ein.

Eine Reihe von Wachstums- oder neurotrophen Faktoren können das Überleben glaukomatöser Neurone verlängern. Dazu gehören NGF, BDNF, CNTF, bFGF und Neurotrophin 3,4-5. In einer bevorzugten Ausführungsform könnten erfindungsgemäße Pigmentepithelzellen zur Therapie eines Glaukoms verwendet werden, die als therapeutisches Gen das Gen für NGF, BDNF, CNTF, bFGF und/oder Neurotrophin 3,4-5 enthalten. Diese Faktoren könnten dann das Überleben durch die Aktivierung spezieller Stoffwechselwege regulieren. Viele dieser Faktoren haben eine kurze Halbwertszeit. Die stabile Expression dieser Faktoren ist folglich von erheblicher therapeutischer Bedeutung.

Die Diabetische Retinopathie entsteht bei Diabetes mellitus infolge einer Glykosylierung von Proteinen durch eine Verdickung der Basalmembran der Gefäßendothelien. Sie ist die Ursache film eine frühzeitige Gefäßsklerose, und die Bildung von Kapillaraneurysmen. Diese Gefaßveränderungen führen in Verlauf von Jahren zur Diabetischen Retinopathie. Die Gefäßveränderungen rufen eine Minderperfusion von Kapillargebieten hervor. Dies führt zu Lipoidablagerungen (harte Exsudate) und zu einer Vasoproliferation. Der klinische Verlauf kann bei Patienten mit Diabetes mellitus verschieden sein. Beim Altersdiabetes (Typ II Diabetes) treten zunächst Kapillaraneurysmen auf. Danach erscheinen aufgrund der verschlechterten Kapillarperfusion harte und weiche Exsudate und fleckförmige Blutungen im Netzhautparenchym. In späteren Stadien der Retinopathia diabetica sind die Verfettungen kranzförmig um die Makula angeordnet (Retinitis circinata). In Verbindung mit diesen Veränderungen kommt es häufig zu ein Ödem am hinteren Augenpol. Wenn das Ödem die Makula einbezieht, ist das Sehvermögen akut hochgradig vermindert. Das Hauptproblem beim Typ-1-Diabetes ist die Gefäßproliferation im Bereich des Augehhintergrunds. Standardtherapie ist die Laserkoagulation der betroffenen Bereiche des Augenhintergrundes. Zunächst wird die Laserkoagulation fokal in den betroffenen Netzhautarealen durchgeführt. Bei Fortbestehen der Exsudate wird die Laserkoagulation flächenhaft ausgedehnt. Die Netzhautmitte mit der Stelle des schärfsten Sehens, also die Makula und das papillomakuläre Bündel, kann nicht koaguliert werden, da es durch den Eingriff zur Zerstörung der für das Sehen wichtigsten Netzhautteile käme. Sind bereits Proliferationen entstanden, müssen oft die Herde an der Basis der Proliferationen sehr dicht gesetzt werden. Dabei wird die Netzhaut flächenhaft zerstört. Es entsteht ein entsprechender Gesichtsfeldausfall. Beim Typ-1-Diabetes ist die rechtzeitige Laserkoagulation häufig die einzige Chance, die Patienten vor der Erblindung zu bewahren.

Ein Beispiel für eine genetisch bedingte Erkrankung des Pigmentepithels ist die autosomal-rezessive schwere retinale Dystrophie mit Beginn in der Kindheit, die durch Mutation in dem RPE65 Gen verursacht wird (Gu SM, Thompson DA, Srikumari CR, Lorenz B, Finckh U, Nicoletti A, Murthy KR, Rathmann M, Kumaramanickavel G, Denton MJ, Gal A (1997) Mutations in RPE65 cause autosomal recessive childhood-onset severe retinal dystrophy. Nat Genet 17: 194-197). Von dem Einbringen des RPE65 Gens mit Hilfe eines adenoviralen Vektors mit großer DNA Kapazität ist eine Korrektur des pathologischen Phänotyps zu erwarten.

Weiterhin war völlig überraschend, daß Pigmentepithelzellen des Auges auch in das ZNS transplantiert werden können. Es konnte in Rahmen der vorliegenden Erfindung gezeigt werden, daß Pigmentepithelzellen den Beobachtungszeitraum von 5 Wochen überlebten. Die histologische Untersuchung ergab keinen Anhaltspunkt für die Induktion einer Schädigung neuraler Zellen. Stattdessen konnte beobachtet werden, daß die Pigmentepithelzelen intensive Kontakte mit Neuronen bildeten.

Bisher wurden eine Reihe von verschiedene Zelltypen im Tierexperiment oder bei Patienten mit Morbus Parkinson im Rahmen von klinischen Studien eingesetzt: Beispiele sind fetale Zellen, gewonnen aus Gehirnen von menschlichen Feten. Fetale Zellen aus dem ventralen Mittelhirn oder dopaminerge Neurone wurden bereits in klinischen Studien bei über 300 Patienten mit Morbus Parkinson transplantiert (zur Übersicht siehe Alexi T, Borlongan CV, Faull RL, Williams CE, Clark RG, Gluckman PD, Hughes PE (2000) Neuroprotective strategies for basal ganglia degeneration: Parkinson's and Huntington's diseases. Prog Neurobiol 60: 409-470). Eine Reihe von verschiedenen Zelltypen, darunter auch nicht neuronale Zellen z.B. Zellen aus der Niebennierenrinde, Sertolizellen aus den Keimdrüsen oder Glomuszellen aus den Carotiden, Fibroblasten oder Astrozyten wurden bei Patienten mit Morbus Parkinson oder Tiermodellen verwendet, mit dem Ziel Dopamin spontan oder nach Gentransfer (Alexi et al. 2000, supra) zu substituieren. Die Überlebensrate transplantierter fetaler dopaminerger Neurone liegt bei 5-8 %, was bereits eine geringe Verbesserung der klinischen Symptome verursachte (Alexi et al. 2000, supra).

In den letzten Jahren wurden neuronale Stammzellen aus Gehirnen von adulten Vertebraten isoliert, in-vitro expandiert und ins ZNS reimplantiert, worauf sie sich in reife Neurone differenzierten. Ihre Funktion im ZNS bleibt aber ungewiß. Neuronale Vorläuferzellen wurden auch für den Gentransfer benutzt (Raymon HK, Thode S, Zhou J, Friedman GC, Pardinas JR, Barrere C, Johnson RM, Sah DW, (1999) Immortalized human dorsal root ganglion cells differentiate into neurons with nociceptive properties. J Neurosci 19: 5420-5428). Schwannsche Zellen, die NGF und GDNF überexprimierten, zeigten eine Neuroprotektion in Parkinsonmodellen (Wilby MJ, Sinclair SR, Muir EM, Zietlow R, Adcock KH, Horellou P, Rogers JH, Dunnett SB, Fawcett JW (1999) A glial cell line-derived neurotrophic factorsecreting clone of the Schwann cell line SCTM41 enhances survival and fiber outgrowth from embryonic nigral neurons grafted to the striatum and to the lesioned substantia nigra. J Neurosci 19: 2301-2312).

Der Vorteil der Pigmentepithelzellen gegenüber den bisher verwendeten Zellen besteht darin, daß sie insbesondere bei der Verwendung körpereigene (autologe) Zellen nicht vom Immunsystem abgestoßen werden und damit erwartungsgemäß eine sehr hohe Überlebensrate haben. Außerdem ersetzten sie natürliches Melaninpigment, welches in der Substantia nigra von Parkinson Patienten verloren geht. Dieses Melanin kann freies Fe⁺⁺ detoxifizieren und damit den Krankheitsverlauf günstig beeinflussen.

Ein anderer Gegenstand der vorliegenden Erfindung ist daher die Verwendung von erfindungsgemäßen Pigmentepithelzellen zur Therapie von Nervenerkrankungen, insbesondere einer Erkrankung des Nervensytems, bevorzugt des ZNS, vor allem von Morbus Parkinson.

Ein Beispiel für eine häufige Erkrankung des ZNS ist der Morbus Parkinson, bei der es sich um ein chronisch-degenerative Erkrankung des Gehirns handelt. Ursache der Erkrankung ist die Degeneration von spezialisierten neuronalen Zellen im Bereich der Basalganglien. Wegen des Untergangs dopaminerger Neurone kommt es beim Patienten mit Morbus Parkinson zur verminderten Synthese von Dopamin, einem wichtigen Neurotransmitter. Eine Standardtherapie ist die medikamentöse Therapie mit L-Dopa. L-Dopa wird in den Basalganglien zu Dopamin metabolisiert und übernimmt dort die Funktion des fehlenden endogenen Neurotransmitters. Nach einigen Jahren verliert die L-Dopa Therapie jedoch ihre Wirksamkeit.

Pigmentepithelzellen produzieren bereits spontanerweise einige Faktoren, die eine neuroprotektive Wirkung haben. Beispiele für solche Faktoren, die beispielsweise von IPE Zellen produziert werden, sind "nerve growth factör" (NGF), "ciliary neurotrophic factor" (CNTF), "basic fibroblast growth factor" (bFGF) oder Faktoren mit angiogener Wirkung wie zum Beispiel "vascular endothelial growth factor" (VEGF) oder "platelet derived growth factors A und B" (PDGF A+B). Ein Beispiel für einen neurotrophen Faktor, der in den genetisch modifizierten IPE Zellen nach einem Gentransfer produziert werden kann, ist der "glial cell derived neurotrophic factor" (GDNF).

Außerdem kann die natürliche Schutzfunktion der Pigmentepithelzellen ausgenutzt werden. Bei Morbus Parkinson könnten transplantierte IPE Zellen durch die antioxidative Wirkung ihrer Melaningranula eine neuroprotektive Wirkung entfalten. Dieses könnte durch die Fähigkeit des Melanins und seiner Vorstufen verursacht werden, Fe²⁺ und andere toxische Substanzen (z.B. Ca²⁺) zu binden und damit dem Zellzytoplasma zu entziehen (Hill HZ (1992) The function of melanin or six blind people examine an elephant. Bioessays 14: 49-56) bzw. sehr stark antioxidativ zu wirken. IPE Zellen haben einen hohen Melaningehalt und fahren auch dann fort, Melanin zu bilden, wenn sie in der Netzhaut, dem subretinalen Raum oder dem ZNS lokalisiert sind. Ein eindeutiger Hinweis hierfür ist das Vorliegen von zahlreichen frühen Melanogenesestadien (Prämelanosomen), die durch elektronenmikroskopische Untersuchungen nachgewiesen werden konnten Melanin hat antioxidative Eigenschaften, schützt vor Lipidperoxidation, kann direkt Sauerstoffradikale binden (Hill HZ (1992) The function of melanin or six blind people examine an elephant. Bioessays 14: 49-56) und kann die Bildung neuer Sauerstoffradikale durch Bindung von Metallkationen verhindern (Memoli S, Napolitano A, d'Ischia M, Misuraca G, Palumbo A, Prota G (1997) Diffusible melanin-related metabolites are potent inhibitors of lipid peroxidation. Biochim Biophys Acta 1346: 61-68). Bringt man stark pigmentierte Irispigmentepithelzellen in Gewebe mit hohem oxidativen Stress, zum Beispiel in die Substantia nigra von Patienten mit Morbus Parkinson, oder in die Papille von Glaukompatienten, oder in die Nähe der Makula von AMD Patienten, dann tritt bereits durch die Anwesenheit des Melanins in den IPE Zellen eine neuroprotektive Wirkung ein.

Ein Beispiel für ein Protein mit gutem therapeutischen Potential für die Therapie von Patienten mit Morbus Parkinson ist glial cell derived neurotrophic factor (GDNF) ein Überlebensfaktor für dopaminerge Neurone. GDNF wirkt bereits in picomolarer Konzentration auf die Uberlebenrate und das Wachstum dopaminerger Neurone aus dem embryonalem Hirn. Tierexperimentelle Untersuchungen haben gezeigt, daß ein direkter Gentransfer in die Substantia nigra einer GDNF Expressionskassette unter Verwendungen verschiedener Vektoren (adenovirale Vektoren der ernsten Generation, AAV Vektoren oder lentivirale Vektoren) dopaminerge Neurone im 6-OHDA Rattenmodel schützen konnte (Mandel RJ, Spratt SK, Snyder RO, Leff SE (1997) Midbrain injection of recombinant adeno-associated virus encoding rat glial cell line-derived neurotrophic factor protects nigral neurons in a progressive 6-hydroxydopamine-induced degeneration model of Parkinson's disease in rats. Proc Natl Acad Sci U.S.A. 94: 14083-14088). In einer bevorzugten Ausführungsform wird GDNF in autologen IPE Zellen nach Transduktion mit einem adenoviralen Vektor großer DNA-Kapazität exprimiert. Die derart genetisch modifizierten Zellen werden dann stereotaktisch an den Wirkort, zum Beispiel in das Striatum, implantiert. In einer weiteren Ausführungsform wird das für das therapeutische Protein kodierende Gen reguliert exprimiert; zum Beispiel durch Verwendung eines zellspezifischen Promotors. In einer bevorzugten Ausführungsform wird das therapeutische Gen regulierbar, zum Beispiel durch Verwendung eines Tetrazyklin regulierbaren Systems, exprimiert.

In einer bevorzugten Ausführungsform dieser Erfindung werden Pigmentepithelzellen an den gewünschten Ort im ZNS implantiert. Die Zellen können beispielsweise durch Injektion, zum Beispiel im Rahmen einer stereotaktischen Operation, an den gewünschten Wirkort gebracht. Hierdurch ist es möglich spezifische therapeutische Moleküle vor Ort zu produzieren.

In einer besonders bevorzugten Ausführungsform werden autologe Pigment-epithelzellen verwendet. Die Verwendung von autologen Pigmentepithelzellen hat folgende Vorteile gegenüber anderen Zelltypen: es kommt nicht zu Abstoßungsreaktionen, da die verwendeten Pigmentepithelzellen vom Patienten selbst stammen; es handelt sich entwicklungsgeschichtlich um neuroepitheliale Zellen, die mit den Zellen des Gehirns histogenetisch verwandt sind.

In einer bevorzugten Ausführungsform dieser Erfindung werden erfindungsgemäße Pigmentepithelzellen, insbesondere autologe IPE Zellen, zur Therapie von Erkrankungen des ZNS, wie zum Beispiel dem Morbus Parkinson verwendet.

Die erfindungsgemäßen Pigmentepithelzellen können desweiteren in der Transplantation verwendet werden. In einer ausführungsform der Erfindung können die erfindungsgemäßen Pigmentepithelzellen durch Transplantation in das Auge ihre therapeutische Wirkung entfalten.

Die Pigmentepithelzellen können beispielsweise in die Aderhaut transplantiert werden und dort durch die Produktion von Pigmentepithel-endogener Faktoren oder durch die Produktion von therapeutischen Molekülen nach genetischer Modifikation mit einem adenoviralen Vektor großer DNA-Kapazität einen therapeutischen Effekt ausüben.

Die Pigmentepithelzellen können aber auch im Bereich der Papille eingesetzt. Im Rahmen der vorliegenden Erfindung konnte gezeigt werden, daß die Zellen nach Injektion in den posterioren Teil des Glaskörpers in den Papillenkopf einwandern und sich dort im Gewebeverband integrieren. Dies eröffnet die Möglichkeit zur Therapie von Erkrankungen, die sich beispielsweise im Papillenkopf manifestieren.

Die Pigmentepithelzellen können auch in den Glaskörper, insbesondere durch einen transskleralen Zugang in den posterioren Teil des Glaskörpers, injiziert werden. Es konnte durch fundoskopische Beobachtung bei RCS Ratten gezeigt werden, daß die IPE Zellen über einen Beobachtungszeitraum von 2 Monaten vollständig auf der Papille wiedergefunden werden. Histologisch war erkennbar, daß die IPE Zellen in den Papillenkopf eingewandert waren und dort intensiven Kontakt zu den Blutgefäßen und Axonen bildeten. Ultrastrukturell zeigt sich keinerlei Schädigung neuronaler Zellen oder Proliferation von IPE Zellen im Glaskörper. Diese Ausführungsform der Erfindung ermöglicht beispielsweise einen direkten Zugang zum Papillenkopf. Dabei konnen mit oder ohne genetische Modifikation neuroprotektive Mediatoren in oder in unmittelbarer Nähe der Papille freigesetzt oder aktiviert werden.

Des weiteren wird die Verwendung von adenoviralen Vektoren großer DNA-Kapazität zur genetischen Modifikation von Pigmentepithelzellen in vivo beschrieben. Wie in den Beispielen gezeigt, führt die in vivo Transduktion von RPE Zellen durch subretinale Injektion mit einem adenoviralen Vektor großer DNA-Kapazität zu einer überraschend stabilen Expression von mindestens 6 Monaten.

Ein weiterer Gegenstand der vorliegenden Erfindung betrifft ein Arzneimittel oder Diagnostikum enthaltend eine erfindungsgemäße Pigmentepithelzelle sowie geeignete Hilfs- und/oder Zusatzstoffe. Geeignete Hilfs- und Zusatzstoffe, die beispielsweise der Stabilisierung oder Konservierung des Arzneimittels oder Diagnostikums dienen, sind dem Fachmann allgemein bekannt (siehe z. B. Sucker H et al. (1991) Pharmazeutische Technologie, 2. Auflage, Georg Thieme Verlag, Stuttgart). Beispiele für solche Hilfs- und/oder Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Die folgende Figur und die Beispiele sollen die Erfindung näher erläutern, ohne sie darauf zu beschränken.

### BESCHREIBUNG DER FIGUR

### Fig.1 Schematisierter Querschnitt durch das rechte Auge.

Das Irispigmentepithel befindet sich auf der Seite der Iris (1), die der Linse (9) zugewandt ist. Die Makula ist der Bereich (ca. 6 mm Durchmesser), der die Fovea (10) unmittelbar umgibt.

| | | | |
|---|---|---|---|
| 1 | Iris | 8 | Pupille |
| 2 | Ziliarkörper | 9 | Linse |
| 3 | Glaskörper | 10 | Fovea |
| 4 | Sklera | 11 | Papille |
| 5 | retinales Pigmentepithel | 12 | hintere Augenkammer |
| 6 | Retina | 13 | vordere Augenkammer |
| 7 | Aderhaut | 14 | Hornhaut |

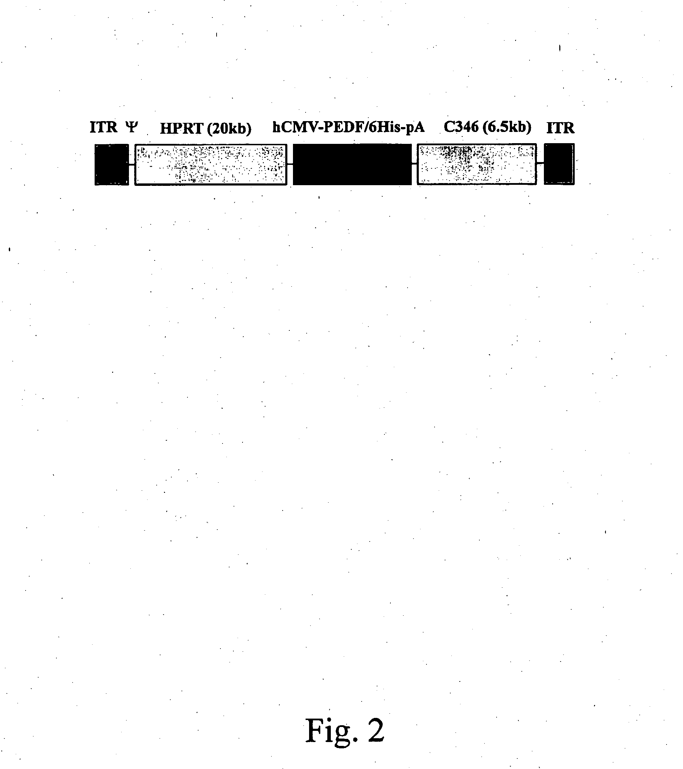

### Fig. 2 Genomische Struktur von HC-Ad.PEDF

Merkmale von links nach rechts: linker Terminus der Adenovirus Typ 5-DNA (nt 1-440) einschließlich des Packungssignals Ψ; 20 kb eines DNA-Füllfragments (stuffer DNA fragment) hergestellt aus den humanen HPRT Locus (Genkartenposition 1777-21729, Locus: HUMHPRTB); hCMV Promoter und PEDF cDNA mit einem C-terminalen 6-His-Tag; SV40 poly A; 6,5 kb humanes Fragment von C346 (Cosmidkartenposition 10205-16750, Locus: HUMDXS455A); rechter Terminus des Adenovirus Typ 5 (nt 35818-35935).

### BEISPIELE

### 1. Isolierung der Zellsheets

Zur Isolierung der IPE-Zellen in Zellverbänden (Zellsheets) wurden Iridektomien frisch aus dem Operationssaal nach einer Trabekulektomie oder einer basalen Iridektomie abgeholt, in F12 Medium ((HAM) mit L-Glutamin, Gibco, Life Technologies, Paisley Scotland) gebracht und direkt weiterverarbeitet. Basale Iridektomien von Glaukompatienten oder Irisstücke von Ratten oder Schweinen wurden mit Accutase (Cat. No. L11-007, PAA Laboratories) in Dulbeccco's PBS mit 0,5 mmol/l EDTA x Na für 15-20 min behandelt. Das Gewebe, welches mit einer Iridektomie gewonnen werden kann, hat eine Fläche von ca. 3,5 mm² und enthält ca. 20000 IPE Zellen. Die Zellagen werden sehr behutsam mit F12 Medium auf- und nieder pipettiert und auf Polystyrene ausplattiert. Es konnten die IPE Zellen vollständig als doppelte Zellage mit intakter Basalmembran unter der Stereolupe vom Stroma gelöst werden, was durch elektronenmikroskopische Untersuchung nachgewiesen werden konnte. Durch Inkubation mit 0.1 U/ml Chondroitinase ABC (Sigma) und 2.4 U/ml Heparinase (Sigma) in PBS bei pH 7.4 für 2 Stunden bei 37 Grad Celsius konnte diese Basalmembran vollständig entfernt werden. Anschließend konnten die Zellsheets durch erneute 5 minütige Accutaseinkubation in Einzelzellen zerlegt werden.

Zur Isolierung der RPE-Zellen in Zellverbänden (Zellsheets) wurden in menschlichen Augen in der Peripherie nach lokaler Retinotomie autologe RPE Zellsheets und Einzelzellen mechanisch losgelöst und mit einer Kanüle abgesaugt. Lokal konnten 50.000 periphere RPE Zellen entnommen werden ohne dass die Patienten später über unangenehme gravierende Gesichtfeldausfälle klagen. Die Entnahme von je 50.000 RPE Zellen konnte an mehreren peripheren Stellen des Auges erfolgen.

### 2. Vermehrung von IPE und RPE Zellen durch Kultivation auf Fibroblasten

IPE oder RPE Zellen, die aus Iridektomien oder Augen von Organspendern gewonnen wurden, wurden auf Fibroblasten (3T3 Fibroblastenzelllinie der Maus), die als "feeder layer" dienen, in F12 Medium kultiviert. Die Zellen werden innerhalb von einem Tag auf den Fibroblasten adhärent und beginnen zu proliferieren. Die Zahl der Zellen verdrei- bis vervierfachte sich auf den Fibroblasten innerhalb von 3 Tagen. Die Fibroblasten wurden zuvor mit 40 µg/ml Mitomycin C behandelt, so dass sie nach spätestens 10 Tagen absterben. Nach diesem Zeitpunkt erhält man eine Reinkultur an Pigmentepithelzellen.

### 3. Injektion der IPE Zellen

IPE Zellen wurden als Einzellzellsuspension wie unter 1 beschrieben isoliert. Ein Tiermodell für die altersabhängige Makuladegeneration und für die retinale Degeneration, die durch einen spezifischen Phagozytosedefekt und Degeneration des RPE bedingt ist, ist die Royal College of Surgeons Rat (RCS Ratte). Dystrophen RCS Ratten (18 Tage alt) wurden in Ketanest/Nembutal Narkose die obere Bindehaut durch einen 4 mm langen Schnitt 4 mm hinter dem Limbus eröffnet. Dabei wurde die limbusnahe Bindehaut mit einer Kolibripinzette festgehalten.

### 3.1. Subretinale Injektion

Bei der subretinalen Injektion wurden mit einer 26 Gauge Kanüle Sklera, Aderhaut und retinales Pigmentepithel in Höhe des Äquators bis auf den Glaskörper durchstochen. Eine Hamiltonspritze mit stumpfer 32 Gauge Kanüle wurde 2-3 mm tangential zwischen Retina und RPE nach anterior eingeführt. Es wurden 60000 IPE Zellen in 0.5 µl Zellkulturmedium (F 12 (HAM) mit L-Glutamin, Gibco, Life Technologies, Paisley Scotland) injiziert. Nach Ablauf der Beobachtungszeit von 6 und 8 Monaten wurden die RCS Ratten getötet. Die Augen wurden enukleiert. Die Hornhäute wurden entfernt und die verbleibenden Teile der Augen wurden in 3 % Glutaraldehyd fixiert. Areale mit transplantierten Iriszellen konnten leicht an der Pigmentierung erkannt werden, wurden herausgeschnitten und für elektronenmikroskopischen Untersuchungen nach Routineprotokoll eingebettet. Ultrastrukturell waren überlebende, d.h. morphologisch intakte IPE Zellen im subretinalen Raum bis zu 8 Monaten nach Transplantation nachweisbar. Überlebende Photorezeptoren mit Innensegmenten aber ohne Außensegmente waren bis zu 6 Monaten nach subretinaler Transplantation vorhanden.

### 3.2 Injektion in den Glaskörper

Bei der Injektion in den Glaskörper erfolgte die Injektion an gleicher Stelle wie subretinal in 6 Augen. Allerdings wurde die Kanüle wie eine Kreisbogensekante 1-2 mm tief in den Glaskörper eingeführt. Die Injektion von 60000 IPE Zellen erfolgte in der Nähe der Papille. Im Beobachtungszeitraum von 2 Monaten blieb der Glaskörper und die Linse klar. Die IPE Zellen versammelten sich in allen 6 Augen makroskopisch oder funduskopisch sichtbar auf der Papille. Die Histologie zeigte, dass die IPE Zellen in den Papillenkopf einwanderten. Die Zellen waren stark pigmentiert und es gab keine Anzeichen von Zellschädigung oder Proliferation.

### 3.3. Injektion in die Aderhaut

Bei der Injektion in die Aderhaut wurde der gleiche Injektionsort wie subretinal gewählt. Die Sklera wurde mit einem spitzen Skalpell durch einen 1 mm langen Schnitt durchtrennt bis die Aderhaut sichtbar wurde. Die Kanüle wurde senkrecht zum Augapfel auf die Schnittstelle gestellt und 60000 IPE Zellen in 0.5 µl F12 Medium wurden in die Aderhaut injiziert. In die Aderhaut transplantierte IPE Zellen führten in 15 Augen verglichen mit 6 unbehandelten Augen zu einem Überleben von Photorezeptoren bis zu 6 Monaten. Sowohl die Anzahl der überlebenden Photorezeptoren/ mm Retina (p=0.020) als auch die maximale Kernhöhe (p=0.019) waren im Mann-Whitney-Test signifikant unterschiedlich gegenüber den unbehandelten Augen (Tabelle 1).

**Tabelle 1: Median, 25 ste und 75 ste Percentile der Anzahl der noch vorhanden Photorezeptorzellkerne, der maximalen Dicke der Photorezeptorschicht in Semidünnschnitten 6 Monate nach Transplantation von IPE Zellen in die Aderhaut sind angegeben.**

| | **Anzahl Augen** | **der Photorezeptor Kerne [mm⁻¹]** | **Maximale Höhe der Photorezeptorzellkerne** |
|---|---|---|---|
| | | | |
| **IPE-Transplantation** | **15** | | |
| **Median** | | **12.3** | **1.0** |
| **25^{th} Percentile** | | 0.0 | 0.0 |
| **75^{th} Percentile** | | 45.5 | 3.0 |
| | | | |
| **Kontrolle** | **6** | | |
| **ohne Behandlung** | | | |
| **Median** | | **0.0** | **0.0** |
| **25^{th}Percentile** | | 0.0 | 0.0 |
| **75^{th} Percentile** | | 0.0 | 0.0 |
| **Mann-Whitney-Test** | | | |
| **P value** | | **0.020** | **0.019** |

### 4. Injektion der IPE Zellen der Ratte in das ZNS

Bei der stereotaktische Implantationsmethode wurden Wistar Ratten durch 1 ml/ 100 g Körpergewicht Avertin (2g Tribromethanol 3,3,3(trocken), 1 g Pentanol (flüssig), 8 ml 100% iger Ethanol und 90 ml NaCl 0,9%) in Form einer intraperitonealen Injektion narkotisiert. Der Schädel wurde präzise reproduzierbar an drei Knochenpunkten, den äußeren Gehörgängen und der Maxilla fixiert, so daß das Calvarium in Höhe des Bregmas waagerecht stand. Nach der medialen fronto-okzipitalen, 1,5 cm langen Hautinzision, wurde das Periost abgeschoben, um freie Sicht auf die Suturen der Schädelkalotte zu haben, die als Bezugspunkt für die stereotaktischen Koordinaten dienten. Die Koordinaten wurden anhand des Atlas von Praxinos und Watson (Praxinos G, Watson C. The rat brain in stereotactic coordinates. 1986;2 end Dr., Academic Press, Sydney) ermittelt:

Die Punktionsstelle war vom Bregma 1,5 mm nach frontal und 2mm nach rechts parietal. 4,5 mm in der Tiefe liegt der obere Anteil des Striatums. Das Bohrloch mit einem Durchmesser von etwa 0.5 mm wurde an der entsprechenden Position mit einem Präzisions-Wellenbohrer (Proxxon, Minimot 40IE) unter Vermeidung einer Duraläsion angebracht. 5-10 µl der Zellsuspension wurden mit einer 25 µl N-702-N Hamilton-Spritze mit fixierter Nadel durch dieses Bohrloch in einer Injektionstiefe von 5 mm, ab der Duraoberfläche gemessen, eingebracht. Es wurden 60000 IPE Zellen von Long Evans Ratten in die Striata von je 4 Wistar Ratten injiziert. Bevor die Nadel herausgezogen wurde, wartete man 2 min, damit die Zellsuspension im Gewebe abdiffundieren und der lokal entstandene Druck abgebaut werden konnte. Anderenfalls war zu befürchten, daß Zellen der zurückgezogenen Nadel in den Punktionskanal oder in die darüber liegenden Gewebeabschnitte folgen hätten können. Aus dem gleichen Grunde wartete man, nachdem man die Nadel 4 mm zurückgezogen hatte, nochmals 30 sec. bevor sie ganz entfernt wurde. Anschließend wurde eine Hautnaht angefertigt.

Nach 5 Wochen wurden die Gehirne mit 3 % Glutaraldehyd in Cacodylatpuffer perfusionsfixiert. Pigmentierte Areale aus den Striatum wurden herausgeschnitten und für die Elektronenmikroskopie eingebettet.

Makroskopisch konnten die transplantierten Areale leicht durch die Pigmentierung erkannt werden. Unter dem Elektronenmikroskop hatten die IPE Zellen intakte Mitochondrien und Plasmamembranen. Sie waren stark pigmentiert, enthielten Melanogenesestadien und bildeten Kontaktzonen mit Neuronen. Die IPE Zellen wurden stets einzeln ohne Kontakte zu anderen IPE Zellen angetroffen. Sie wurden auch 3-4 mm vom Stichkanal entfernt angetroffen, was eine aktive Wanderung der Zellen vermuten läßt. Die Neurone, die an die IPE Zellen angrenzten, waren morphologisch intakt. Immunkompetente Zellen (Makrophagen, Lymphozyten) wurden nicht beobachtet.

### 5. Genetische Modifikation

Vereinzelte und adhärente Ratten- bzw. menschliche IPE-Zellen wurden in vitro mit 20, 50 und 100 MOI (multiplicity of infection) des adenoviralen Vektors mit grosser DNA Kapazität HC-Adenovirus "HC-AdFK7", der das EGFP (enhanced green fluorescent protein) unter der Kontrolle des humanen CMV (cytomegalovirus)-Promotors als Transgen trägt, transduziert. Dazu wurden 80% konfluente Zellkulturen in F12 komplettem Medium mit der entsprechend verdünnten Virusstammlösung über 24 Stunden bei 37 °C inkubiert. Das Medium wurde am nächsten Morgen gewechselt. Die Expression des Transgens wurde nach 24, 48, 72 Stunden und dann jede weitere Woche unter einem Fluoreszenzmikroskop mit FITC-Filter als grüne Fluoreszenz innerhalb der Zellen überprüft.

Schon 24 Stunden nach der Transduktion war eine zarte Fluoreszenz sichtbar, die sich in den nächsten Tagen deutlich verstärkte. Die menschlichen IPE Zellen waren bei 100 MOI zu 100% transduziert, die Rattenzellen bei 20 MOI zu 80% und bei 50 und 100 MOI zu 100% transduziert. Eine Expression war bis zu einer Zeitspanne von 8 Wochen und länger in vitro nachweisbar.

In einem weiteren Experiment wurde der adenovirale Vektor großer DNA Kapazität AdhCMV.PEDF konstruiert. Dieser Vektor exprimiert die menschliche PEDF cDNA unter der Kontrolle des humanen CMV Promotors. Zusätzlich ist das PEDF Protein durch ein Poly-Histidin Epitop, das als Fusionsprotein mit dem PEDF exprimiert wird, markiert. Dieser Vektor wurde nach einem Standardverfahren (Schiedner G, Morral N, Parks RJ, Wu Y, Koopmans SC, Langston C, Graham FL, Beaudet AL, Kochanek S (1998) Genomic DNA transfer with a high capacity adenovirus vector results in improved in vivo gene expression and decreased toxicity. Nature Genetics 18: 180-183) in Cre-Rekombinase exprimierenden 293 Zellen produziert und durch CsCl Dichtegradienten Zentrifugation aufgereinigt.

Cytokeratin-positive humane IPE Zellen der 2. Passage wurden mit dem HC-Ad.CMV.PEDF Vektor transduziert. Dazu wurden 80% konfluente Zellkulturen in F12 komplettem Medium mit der entsprechend verdünnten Virusstammlösung über 24 Stunden bei 37°C inkubiert. Das Medium wurde am nächsten Morgen gewechselt. Die Expression des Transgens und die Sekretion des PEDF in den Kulturüberstand wurde nach 72 Stunden mit Hilfe spezifischer anti-Polyhistidin-Antikörper im ELISA überprüft. Die Kulturüberstände enthielten 150 ng PEDF/ml. Dies entspricht einer Produktion von 60 pg PEDF pro 1000 Zellen/72 Stunden. Durch Verwendung eines weiteren Antikörpers, der spezifisch das menschliche PEDF Protein erkennt, konnte in einem weiteren ELISA nachgewiesen werden, dass menschliche IPE Zellen im Gegensatz zu menschlichen RPE Zellen spontanerweise kein PEDF produzieren.

### IPE sheet Transfektion

Neben Einzelzellen konnten auch Zellsheets transfiziert werden. Nach enzymatischer Entfernung der Basalmembranen waren IPE Zellsheets transfizierbar. Mit intakter Basalmembran waren die Zellsheets nicht transfizierbar, was mit PCR nachgewiesen wurde.

Zur Transfektion von Zellsheets wurden Schweineaugen aus dem Schlachthof direkt nach der Schlachtung der Tiere in das Labor gebracht und weiterverarbeitet. Der Vorderabschnitt wurde ca. 2 mm hinter dem Limbus zirkulär abgetrennt. Von posterior wurde dann die Iris stumpf abpräpariert und für 15 min bei 37 °C in 1 ml Accutase inkubiert. Mit einer im Feuer gebogenen Glaspipette wird dann das IPE vom Stroma der Iris gelöst. Eine Schätzung der Fläche der einzelnen erhaltenen IPE Zellsheets ergab zwischen 40.000 und 70.000 IPE Zellen pro Zellsheet.

Die IPE sheets wurden mit 200 MOI des EGFP-exprimierenden adenoviralen Vektors HC-AdFK7 über 24 h inkubiert. Nach 24 Stunden wurde das Medium gewechselt. Die IPE sheets wurden in F12 kompl. Medium für 6 Tage zwischenkultiviert und auf EGFP Fluoreszenz hin untersucht, diese konnte jedoch aufgrund der Morphologie der Zellen mit sehr dicht konzentrierten Melaningranula nicht sicher dargestellt werden. Daraufhin wurde mittels des QIAmp DNA Mini Kit (Qiagen) aus den Zellen DNA gewonnen. Die Angaben des Herstellers wurden befolgt. Mit den Primern prod1, der im Bereich des CMV Promotors und prod2, der im Bereich der Sequenz des EGFP an die DNA des HC-AdFK7 bindet, wurde mittels PCR das Transgen nachgewiesen. Prod1 und prod2 produzierten bei erfolgreich transduzierten Zellen ein PCR-Produkt von ca. 700 Basenpaaren Länge. Als Positivkontrolle diente ein Plasmid pFK7 mit dem gleichen insert wie es in HCAdFK7 zu finden ist.

### 6. Transplantation genetisch modifizierter Zellen subretinal

Mit dem HC-AdFK7 Vektor transduzierte IPE Zellen von Long Evans Ratten (albinotisch) wurden subretinal in 8 Augen von 8 Wistar Ratten transplantiert, nach der gleichen Methode wie für nicht transfizierte IPE Zellen beschrieben.

Vier Augen, in die IPE Zellen transplantiert worden waren, wurden nach 2 Monaten enukleiert, in Tissue Freezing Medium (Jung, Heidelberg, Germany) eingeschlossen und bei -80°C eingefroren. Kryostatschnitte (7 µm) wurden nach dem Auftauen in Kaisers Glyceringelatine (Merck, Darmstadt, Germany) eingeschlossen und in einem Zeiss Axiophot Lichtmikroskop bei einer Anregungswellenlänge von 400-440 nm und einer Emissionswellenlänge von 470 nm mikroskopiert. Die subretinal transplantierten IPE Zellen zeigten eine deutliche Expression grün fluoreszierender Proteine.

Zur Auswertung mit dem Scanning Laser Ophthalmoscope (SLO) wurden die übrigen 4 transfizierten Ratten nach 14 Tagen bzw. 4 Monaten nach der Transfektion untersucht. Die Tiere wurden mit Ketanest narkotisiert und mit dem Scanning Laser Ophthalmoscope (Rodenstock, München) ausgewertet. Dabei wurde im Fluo-Modus mit dem Infrarotlaser (780 nm) und dem Argongrün- (514 nm) bzw. Argonblaulaser (488 nm) die Netzhaut der Ratten in Mydriasis gescannt. Das Gerät benutzt in diesem Modus einen zur Fluoreszenzbeobachtung des EGFP geeignetes Fluoreszeinsperrfilter. Die Bilder wurden auf S-VHS Video aufgenommen. Die analogen Videobilder wurden auf DV digital umkopiert, von repräsentativen Ausschnitten Bitmaps erstellt und mittels der Software Optimas 6.1 nach Fläche und Intensität ausgewertet.

Innerhalb des Beobachtungszeitraumes von 4 Monaten blieb die Intensität der durch die transfizierten IPE Zellen verursachten Fluoreszenz sowie die Flächenausdehnung der transplantierten Areale im Fundus in allen 4 Augen konstant, d.h. die Expression der transfizierten Gene auf Proteinebene blieb unverändert.

### 7. Subretinale Injektion von freiem Vektor zur genetischen Modifikation von RPE Zellen des Wirtes (in-vivo Gentherapie)

Zur subretinalen Injektion von freiem Vektor in vivo wurden verschiedene Konzentrationen eines HC-Adenovirus "HC-AdFK7", der das EGFP (enhanced green fluorescent protein) Gen unter der Kontrolle eines CMV (cytomegalovirus)-Promotors als Transgen trägt, in Wistar Ratten subretinal injiziert. Die Expression des Transgens wurde mit dem Scanning Laser Ophthalmoscope (Rodenstock, München) ausgewertet. Dabei wurde im Fluo-Modus mit dem Infrarotlaser (780 nm) und dem Argongrün- (514 nm) bzw. Argonblaulaser (488 nm) die Netzhaut der Ratten in Mydriasis gescannt. Das Gerät benutzt in diesem Modus einen zur Fluoreszenzbeobachtung des EGFP geeignetes Fluoreszeinsperrfilter. Die Bilder wurden auf S-VHS Video aufgenommen. Die analogen Videobilder wurden auf DV digital umkopiert, von repräsentativen Ausschnitten Bitmaps erstellt und mittels der Software Optimas 6.1 nach Fläche und Intensität ausgewertet.

Innerhalb des Beobachtungszeitraumes von 6 Monaten blieb die Intensität der durch die transfizierten IPE Zellen verursachten Fluoreszenz sowie die Flächenausdehnung der transplantierten Areale im Fundus in allen 4 Augen konstant, d.h. die Expression der transfizierten Gene auf Proteinebene blieb unverändert. Nach 6 Monaten wurden die Tiere getötet, die Augen in 3 % Glutaraldehyd fixiert. Die Vorderabschnitte der Augen wurden entfernt und die verbleibenden hinteren Augenbecher wurden viergeteilt. Nachdem die Netzhäute entfernt wurden, wurden die Sklera, Aderhaut mit Pigmentepithel unter dem Fluoreszensmikroskop (AxioVert, Zeiss, Oberkochen, Germany) mit dem Anregungsfilter 450-490 nm und Emissionsfilter 520 nm (AF Analysentechnik, Tübingen, Germany) untersucht. Dabei zeigte sich die typisch hexagonale Form transduzierter und EGFP positiver Pigmentepithelzellen.

### 8. Transplantation genetisch modifizierter Zellen in das ZNS

Zur Transplantation genetisch modifizierter Zellen in das ZNS wurden mit dem HC-AdFK7 Vektor transduzierte und EGFP-exprimierende IPE Zellen (60000) aus Long Evans Ratten stereotaktisch wie oben beschrieben in das Striatum von je 4 Wistar Ratten injiziert.

Nach 8 Wochen wurden die Tiere durch Genickbuch in C0₂ Narkose getötet. Die Gehirne wurden herauspräpariert. Pigmentierte Areale mit transplantierten Zellen wurden aus dem Striatum herausgeschnitten und in Tissue Freezing Medium (Jung, Heidelberg, Germany) eingefroren. Die Fluoreszenz, verursacht durch die Expression von EGFP der IPE Zellen, war in Gefrierschnitten 8 Wochen nach der Transplantation in pigmentierten Zellen nachweisbar.

### 9. Verhinderung der chorioidalen Neovaskularisation durch genetisch modifiziert und PEDF exprimierende IPE Zellen in-vivo

IPE Zellen wurden mit dem adenoviralem Vektor großer DNA Kapazität HC-AdFK7, der das EGFP (enhanced green fluorescent protein) Gen unter der Kontrolle eines CMV (Zytomegalovirus)-Promotors als Reportergen trägt, und gleichzeitig mit dem PEDF exprimierendem adenoviralem Vektor großer DNA-Kapazität HC-AdCMV.PEDF in vitro cotransfiziert und nach 6 Tagen in den subretinalen Raum von Long Evans Ratten (60.000 Zellen/Auge) transplantiert (1. Versuchsgruppe). Die PEDF Expressionskassette im adenoviralen Vektor großer DNA Kapazität enthielt zusätzlich zur PEDF kodierenden Sequenz ein Poly-HIS-Epitop zum Nachweis des Proteins mittels eines Anti-HIS-Antikörpers. Eine Woche nach Injektion wurden die Ratten narkotisiert, die Pupillen wurden dilatiert und die Ratten erhielten 3-4 Laserverbrennungen um den Nervus Opticus mit einem blau-grünen Argon-Laser (Coherent, Inc., Santa Clara, CA, USA). Die Energie des Lasers betrug 90 mW für 100 ms und der Strahldurchmesser war 100 µm. Eine zweite Gruppe von Ratten erhielt nur Laserverbrennungen ohne genetische Modifikation durch Zelltransplantation oder freie Vektoren. Nach 16 Tagen wurden die Ratten narkotisiert und erhielten 0,5 ml Liquemin i.p. (Roche, Grenzach-Wyhlen, Deutschland). Die Aorta ascendens wurde kanuliert und nach Eröffnung des rechten Vorhofes wurde das Blut mit 50 ml laktierter Ringerlösung (Stereofundin, Braun, Melsungen, Deutschland) ausgewaschen. Anschließend wurden 20 ml Ringerlösung mit 5mg/ml FITC-Dextran (Sigma Deisenhofen, Deutschland) perfundiert. Die Augen wurden enukleiert, in Höhe des Limbus mit einem Scalpel angestochen und über Nacht in 4 % Paraformaldehyd fixiert. Am nächsten Tag wurde der Vorderabschnitt der Augen bis kurz hinter die Ora serrata durch einen umlaufenden Schnitt abgetrennt. Durch 4 radiale Schnitte wurde der verbleibende Augenbecher in Quadranten geteilt und die Netzhäute wurden entfernt. Quadranten, bestehend aus Pigmentepithel, Chorioidea und Sklera, die Lasemarben enthielten, wurden 4 x 10 min in Tris-Puffer (TBS) und dann 10 min in 0,5 M NH₄Cl (Sigma, Deisenhofen, Deutschland) und 0,25 % Triton (Serva, Heidelberg, Deutschland) inkubiert. Nach zwei weiteren Spülungen wurden die Präparate mit 5 % BSA (Albumin, Bovine Fraktion Sigma, Deisenhofen, Deutschland) inkubiert. Ein Teil der Präparate wurde mit Antikörpern gegen Histidin (Anti-His-Antibody, Qiagen, Hilden, Deutschland) inkubiert, um die Histidinreste im PEDF zu detektieren. Die primären Antikörper wurden mit anti-Maus IgG gekoppelt an den Fluoreszensfarbstoff Cy3 (Rockland, Gilbertsville, PA, USA) sichtbar gemacht.

Ein anderer Teil der Präparate wurde mit anti-Maus CD 31 aus der Ratte (PECAM-1, Pharmingen, San Jose, CA, USA) behandelt, um die Endothelzellen sichtbar zu machen. Es folgte eine 2. Inkubation mit anti-Ratte IgG-Biotin (Amersham, Pharmacia Biotech Europe GmbH, Freiburg, Deutschland) mit anschließender Lokalisation des Biotins durch Fluorolink Cy3 (Amersham Life Sciences, Braunschweig, Deutschland). In einigen Aderhautpräparaten erfolgte eine Doppelmarkierung, von PEDF und Endothelzellen. In diesen Fällen wurde PEDF Expression mit Cy3, wie beschrieben, und der PECAM-Biotin-Komplex mit Streptavidin- Alexa Fluor 350 (MoBiTec, Göttingen, Deutschland) sichtbar gemacht.

Die "Flatmount-Präparate" wurden unter dem Fluoreszenzmikroskop (Axiophot, Zeiss, Oberkochem, Deutschland) ausgewertet.

In Gruppe 1 war in 16 von 19 Lasernarben weder ein Austritt von FITC-Dextran noch ein vermehrtes Auftreten von CD 31 positiven Zellen zu beobachten, wenn PEDF exprimierende IPE Zellen im Abstand von 100-1000 µm von der Narbe anwesend waren. Die Expression von PEDF durch die transplantierten IPE Zellen wurde mit anti -His Antikörpern nachgewiesen.

In der Kontrollgruppe 2 (nur Laserverbrennung) war in 9 von 12 Lasemarben Neovaskularisation vorhanden. Dies wurde erkannt am Austritt von Dextran-FITC in und um den Narbenbereich als auch durch das Vorhandensein von abgeflachten CD 31 positiven Endothelzellen in und um den Narbenbereich.

Damit wurde mit einer funktionellen (Dextran-Leakage) und einer immunologischen Methode (direkter Nachweis der neugebildeten Endothelzellen mit Antikörpern) die Neovaskularisation im gleichen Auge nachgewiesen. Diese "Flatmountpräparate" erlauben die Beurteilung der gesamten Aderhaut. Diese Ergebnisse zeigen, dass die Neovaskularisation durch Transplantation von IPE Zellen, die PEDF von einem adenoviralen Vektor großer DNA Kapazität exprimieren, inhibiert wird.

### 10. Endogene HC-Ad-Vektor-mediierte Produktion von PEDF in IPE- und RPE-Zellen

Die endogene PEDF-Produktion in nicht-transduzierten RPE- und IPE-Zellen wurde bestimmt. RPE-Zellen sekretierten 96 + 9,5 ng/ml PEDF innerhalb von 72 h (n=4), wohingegen endogenes PEDF im Überstand von IPE-Zellen nicht nachweisbar war (Nachweisgrenze 1,56 ng/ml). Daher wurde festgestellt, dass IPE-Zellen im Gegensatz zu RPE-Zellen kein PEDF exprimieren, welches ein Protein ist, von dem angenommen wird, dass es eine Schlüsselrolle in der Homeostase der Retina spielt.

Der Vektor HC-Ad.PEDF wurde so konstruiert, dass er humanes PEDF exprimiert, welches ein C-terminales 6-His-Tag (Fig. 2) trägt. IPE- (Hu, D. N., Ritch, R., McCormick, S. A. & Pelton-Henrion, K. (1992) Invest Ophthalmol Vis Sci 33, 2443-2453) und RPE- (Chang, C. W., Defoe, D. M. & Caldwell, R. B. (1997) Invest Ophthalmol. Vis. Sci. 38, 188-195) Zellkulturen wurden aus Long Evans Ratten hergestellt. Kulturen früher Passagen von choroidalen Endothelzellen (hCEC) (positiv für den v. Willebrand-Faktor) wurden aus Spenderaugen (Hoffmann, S., Spee, C., Murata, T., Cui, J. Z., Ryan, S. J. & Hinton, D. R. (1998) Graefes Arch Clin Exp Ophthalmol 236, 779-784) erhalten. 1 x 10⁶ IPE-Zellen wurden mit 50 MOI HC-Ad.PEDF für 24 h, 48 h oder 72 h transduziert. Nach einem Mediumwechsel wurden die Überstände gesammelt. Das ins Medium abgegebene PEDF wurde mittels ELISA mit Maus-penta-his- (5 µg/ml, Qiagen) und Anti-PEDF-Antikörpern (1 µg/ml, Chemicon) nachgewiesen. Es wurde die endogene PEDF-Produktion von nicht-transduzierten IPE- und RPE-Zellen bestimmt. Standardkurven wurden anhand bekannter Mengen von rekombinantem PEDF hergestellt.

Nach Transduktion der IPE-Zellen mit dem HC-Ad.PEDF-Vektor sekretierten die IPE-Zellen (1 x 10⁶ Zellen/Schale, n=4) PEDF innerhalb von 72 h 250 + 38 ng/ml in den Überstand. Die IPE-Zellen sezernierten nach HC-Ad-mediiertem Gentransfer funktionell aktives PEDF in hohen Konzentrationen.

Um die Effekte von PEDF, welches von IPE-Zellen produziert wurde, auf die hCEC-Funktion zu untersuchen, wurden IPE-Zellen mit 50 MOI HC-Ad.PEDF oder 50 MOI HC-AdFK7 transduziert. Unverdünnte, 1:10 oder 1:100 verdünnte Überstände (konditioniertes Medium = CM), die 72 h nach Mediumwechsel erhalten wurden, wurden den hCEC zugefügt.

Zur Proliferationsanalyse wurden 1 x 10³ hCEC/Well, welche in 96-Wellplatten ausgesät waren, dem CM mit oder ohne 50 ng/ml VEGF (Sigma) bzw. mit oder ohne 1 µg/ml Anti-PEDF-Antikörper ausgesetzt. 5 Tage später wurde die Zellproliferation unter Verwendung des WST-1 Proliferationstests (Roche) bestimmt. Unverdünntes und 1:10 verdünntes CM von IPE-Zellen, die mit HC-Ad.PEDF transduziert waren, reduzierte die VEGF-stimulierte Proliferation von hCEC, ohne einen Effekt auf die unstimulierte Proliferation zu haben. Anti-PEDF-Antikörper (1 µg/ml), welcher in dem CM war, unterband die inhibitorische Wirkung. CM von HC-AdFK7-transduzierten oder von nicht-transduzierten IPE-Zellen beeinflusste weder die stimulierte noch die unstimulierte Proliferation der Zellen.

Zur Analyse der Migration wurden 5 x 10³ hCEC/Ansatz in modifizierten Boyden-Kammern (Tang, S., Gao, Y. & Ware, J. A. (1999) J. Cell Biol. 147, 1073-1084) (FluoroBlock Inserts, Becton Dickinson) in CM mit oder ohne VEGF (50 ng/ml) für 8 h bei 37°C inkubiert. Die migrierten Zellen wurden durch Fluoreszenzkernfärbung (DAPI, Alexis) sichtbar gemacht und in 3 zufällig ausgewählten Feldern/Membran gezählt. CM von HC-Ad.PEDF-transduzierten IPE-Zellen erniedrigte die Migration von hCEC gegenüber angiogenem VEGF von 47,5 + 5,9 auf 14,3 + 5,7 Zellen/Membran (P<0,001). Die Exposition mit CM von HC-AdFK7-transduzierten oder von nicht-transduzierten IPE-Zellen beeinflusste die hCEC-Migration nicht.

Um die Bildung von kapillarähnlichen Röhren als Antwort auf PEDF nachzuweisen, wurden 1 x 10⁴ hCEC/Well in 96-Wellplatten, die mit VEGFenthaltendem ECM-Gel (Chemicon) beschichtet waren, für 24 h in CM kultuviert. CM von mit HC-Ad.PEDF infizierten IPE-Zellen unterdrückte die Bildung neovaskulärer Röhren, wohingegen der Überstand von HC-AdFK7-transduzierten und von nicht-transduzierten IPE-Zellen keinen Effekt hatte.

### 11. Langzeit EGFP-Expression nach subretinaler Transplantation von HC-AdFK7-transduzierten IPE-Zellen

IPE-Zellen wurden mit 50 MOI HC-AdFK7 transduziert oder mit 50 MOI HC-Ad.PEDF und HC-AdFK7 cotransfiziert. Vor der Transplantation wurde das Medium gewechselt, die Zellen 2 mal mit PBS gewaschen und Suspensionen von 5 x 10⁴ Zellen/µl transplantiert (1 µl/Auge). Die Transplantation wurde wie oben erwähnt durchgeführt.

Die EGFP-Fluoreszenz in Wistar-Ratten wurde 7 Tage, 1, 2, 3 und 4 Monate nach der subretinalen Injektion von HC-AdFK7-transduzierten IPE-Zellen durch Laser-Raster-Ophthalmoskopie (Scanning Laser Ophthalomoscopy SLO, Rodenstock, Deutschland) und auf RPE-choroidalen "Flatmounts" (McMenamin, P. G. (2000) Invest Ophthalmol Vis Sci 41, 3043-3048) durch Fluoreszenzmikroskopie (Axioplan, Zeiss, Deutschland) beobachtet. 4 Monate nach Injektion wurden die Gebiete mit den HC-AdFK7-transduzierten IPE-Transplantaten elektronenmikroskopisch untersucht. Die PEDF-Expression in IPE-Transplantaten wurde in mit 4 % Paraformaldehyd fixierten RPE-choroidalen "Flatmounts" und in Paraffinschnitten unter Verwendung eines penta-his-Antikörpers (5 µg/ml) und eines Cy3- (Amersham) oder Peroxidase-konjugierten (Amersham) 2. Antikörpers sichtbar gemacht.

7 Tage nach der Transplantation der HC-AdFK7-transduzierten IPE-Zellen wurden Gebiete mit fleckenartige oder durchgängiger Fluoreszenz durch SLO an den Stellen der Injektion beobachtet. 3 Monate später waren immer noch Gebiete mit heller EGFP-Fluoreszenz in den selben Augen mit vergleichbarer Ausdehnung und Intensität wie nach 1 oder 2 Monaten vorhanden. 4 Monate nach dem Eingriff waren die EGFP-exprimierenden IPE-Zellen in die Wirts-RPE-Schicht integriert, was in den RPE-choroidalen "Flatmounts" beobachtet wurde. Durch elektronenmikroskopische Untersuchungen wurde festgestellt, dass die pigmentierten IPE-Zellen eine 2. Schicht auf dem retinalen Pigmentepithel der Wistar-Wirtsratten bildeten. Die den Transplantaten zugewandten äußeren Segmente der Stäbchen erschienen morphologisch intakt. Daher kann geschlossen werden, dass genetisch modifizierte IPE-Zellen nach Transplantation einen Monolayer bildeten und für mindestens 4 Wochen ohne Nebenwirkungen einen EGFP-Reporter stabil exprimierten.

### 12. Wirkungen von HC-Ad-Vektor-mediierter PEDF-Expression von transplantierten IPE-Zellen in einem Modell der sauerstoffinduzierten retinalen Neovaskularisation

Ein zuvor beschriebenes Modell der Ischämie-induzierten Retinopathie (Smith, L. E., Wesolowski, E., McLellan, A., Kostyk, S. K., D'Amato, R., Sullivan, R. & D'Amore, P. A. (194) Invest Ophthalmol Vis Sci 35, 101-111) wurde unter Verwendung von Wistar-Ratten etabliert. Die Tiere in der normoxischen Gruppe wurden während des gesamten Versuchs bei Raumluft gehalten. Die Ratten der hyperoxischen Gruppe wurden vom 7. (P7) bis zum 12. (P 12) Lebenstag 75 % Sauerstoff ausgesetzt, dann in Raumluft überführt und sofort subretinal mit 1) nicht-transduzierten IPE-Zellen; 2) mit HC-AdFK7 transduzierten IPE-Zellen; 3) mit HC-Ad.PEDF und HC-AdFK7 cotransfizierten IPE-Zellen transplantiert. An P22 wurden die Tiere anästhesiert und mit 50 mg/ml Fluoreszeinisothiozyanat-Dextran (Sigma) wie beschrieben (Smith et al., supra) perfundiert. Die Neovaskularisation wurde an retinalen RPE-choroidalen "Flatmounts" unter Verwendung eines Fluoreszenzmikroskops untersucht. Um die Neovaskularisation zu quantifizieren, wurde die Länge der neu gebildeten gewundenen Blutgefäße, deren Durchmesser mehr als 25 µm betrug, auf der inneren Oberfläche der peripheren Retina durch computergestützte Morphometrie (Openlab software; ImproVision, Inc., Lexington, USA) bestimmt. Zur Quantifizierung wurden die Angiografie-Bilder digitalisiert und anschließend bearbeitet. Die Gefäße wurden entsprechend ihrer Durchmesser klassifiziert. Die Messungen der Gefäßlänge erfolgten in einem Bereich von 800 000 µm²/Auge, welcher bis zu 200 µm entfernt von den transplantierten IPE-Zellen oder in den entsprechenden Regionen der hyperoxischen Kontrollen lag. Zusätzlich zu der Gefäßlänge wurde eine zweite unabhängige Quantifizierung der Neovaskularisation durch Bestimmung der gesamten vaskularisierten Fläche in dem gleichen peripheren Gebiet durchgeführt. Die Fluoreszenzbilder wurden ausgewertet, in dem der Fluoreszenzgrenzwert so gesetzt wurde, dass oberflächliche Retinagefäße, aber nicht die Gefäße des retinalen tiefen Plexus, eingefangen wurden. Vaskularisierte Gebiete wurden auf die gesamte ausgewertete periphere Retinafläche normiert. Darüber hinaus wurden 10 µm dicke serielle Gefrierschnitte durch die Hälfte des Auges angefertigt, µm die epiretinale Lokalisation der oberflächlichen pathologischen Gefäße zu bestätigen. Die Immunfluoreszenzmikroskopie wurde wie oben beschrieben durchgeführt.

Bei P22 wurde an den "Flatmounts" der hyperoxisch behandelten nichttransplantierten Tiere große periphere Gebiete mit erweiterten radialen Gefäßen, Mikroaneurysmen und Hämorrhagien, die typisch für retinale Neovaskularisation sind, beobachtet. Diese waren in Retinae von normoxischen Kontrollen nicht vorhanden. Typische Zeichen von peripherer retinaler Neovaskularisation, die durch vaskuläre Knäuel gekennzeichnet ist, nämlich erweiterte Gefäße mit abnormalem, gewundenem Verlauf auf der inneren Oberfläche der peripheren Retina waren offensichtlich. Die epiretinale Position der neu geformten pathologischen Gefäße wurde in den Gefrierschnitten beobachtet. IPE-Zellen wurden durch EGFP-Expression und ihre konservierte hexagonale Form identifiziert. HC-Ad.PEDF-transduzierte IPE-Implantate verhinderten in den Gebieten der Transplantation die Bildung von pathologisch gewundenen Gefäßen. Die Immunfluoreszenzmikroskopie dokumentierte die Expression von PEDF in den genetisch modifizierten IPE-Transplantaten. Die IPE-Transplantate wurden in der Peripherie, wo typischerweise pathologische Gefäße gebildet werden, nach Hyperoxie-Exposition in diesem Modell (Smith et al., supra) beobachtet. Um die Neovaskularisation zu quantifizieren, wurde die Länge von erweiterten gewundenen Gefäßen auf der inneren Oberfläche der Retina mit einem Durchmesser von mehr als 25 µm in einem Retinagebiet von 800.000 µm²/Auge bestimmt. Die beurteilte Fläche war bis zu 200 µm von den IPE-Transplantaten oder in den entsprechenden peripheren Regionen der hyperoxischen Kontrollen gelegen. Ähnlich wie in den normoxischen Kontrollen waren in der Nähe der PEDF-exprimierenden IPE-Zellen keine pathologischen epiretinalen Gefäße vorhanden. Nicht-transduzierte IPE-Zelltransplantate beeinflussten im Gegensatz zu Hyperoxie-Kontrollen die pathologische Vaskularisation nicht. Zusätzlich zur Gefäßlänge wurde die Größe des vaskularisierten Gebiets (oberflächliche Gefäße) als unabhängiger Parameter der Neovaskularisation bestimmt. Nahe der PEDFexrimierenden IPE-Zellen war die vaskularisierte Fläche auf 0,0153 + 0,0081 µm² Blutgefäße/µm² Gewebe im Gegensatz zu 0,0546 + 0,014 µm² Blutgefäß/µm² Gewebe in retinalen Regionen mit transplantierten nicht-transduzierten IPE-Zellen erniedrigt.

Folglich verhinderte die subretinale Transplantation von PEDF-exprimierenden IPE-Zellen die Neoangiogenese in einem Modell der sauerstoffinduzierten Neovaskularisation.

### 13. Wirkungen von HC-Ad-Vektor-mediierter PEDF-Expression in transplantierten IPE-Zellen in einem Modell der laserinduzierten choroidalen Noevaskularisation (CNV)

IPE-Zellen wurden subretinal in 4 bis 5 Monate alte Long Evans Ratten transplantiert. 6 Tage später wurde eine Laserphotocoagulation (100 µm Bestrahlungsfläche, 0,1 sec. Dauer, 150 mW) unter Verwendung einer Blau-Grün-Einstellung eines kohärenten Novus 2000 Argonlasers (Coherent Inc., USA) nahe bei den transplantierten IPE-Zellen (3 Verbrennungen/Auge) durchgeführt. 10 Tage nach der Transplantation wurde die choroidale Neovaskularisation an RPE-choroidalen "Flatmounts" durch Markierung von Endothelzellen mit einem Ratte-Anti-Maus CD31 (PECAM-1) monoklonalen Antiköper (1 µg/ml; Becton Dickinson) bestimmt. Die Größe der Gebiete, die mit Endothelzellen bedeckt war, wurde durch computergestützte Morphometrie bestimmt. Die Verbrennungen wurden folgendermaßen klassifiziert: Typ I - 100 % Besetzung; Typ II - vollständig frei von Endothelzellen; Typ III sowohl besetzte als auch endothelzellfreie Gebiete vorhanden.

Die Lasernarben wurden in Hellfeldabbildungen durch Verklumpung der Pigmente identifiziert. Die Fluoreszenzgraphie einer Laserverbrennung von nichttransplanierten Laserkontrollen nach Markierung der Endothelzellen mit dem Anti-PECAM-1-Antikörper ergab, dass die Verbrennung (Typ I) komplett (100 %) mit neugebildeten Endothelzellen bedeckt ist. IPE-Transplantate wurden in der Umgebung der Laserverbrennung lokalisiert. Die Existenz von EGFP-exprimierenden IPE-Zellen (cotransduziert mit HC-AdFK7 und HC-Ad.PEDF) in der Nähe von 3 Laserverbrennungen konnte nachgewiesen werden. Diese IPE-Transplantate exprimierten PEDF stark, was durch Färbung mit dem penta-his-Antikörper nachgewiesen wurde. Die Gegenwart von PEDF-exprimierenden IPE-Transplantaten in der nahen Umgebung des Schadens verhinderte die Bildung von neuen Gefäßen in der Verbrennung. Diese Laserverbrennungen wurden als Typ II-Verbrennungen klassifiziert, die vollständig frei von Endothelzellen waren. Darüber hinaus reduzierten PEDF-exprimierende IPE-Zellen (ca. 100) in der Umgebung der Verbrennung die Fläche der Neovaskularisation im Gegensatz zu nicht transplantierten Laserkontrollen. In diesem Fall bedeckten die neu gebildeten Endothelzellen 67 % (47400 µm²) der gesamten Fläche der Verbrennung (70 500 µm²). Die Endothelzellen verblieben am Rand der Narbe und proliferierten nicht und migrierten auch nicht ins Zentrum der Verbrennung. Die Flächen, die innerhalb der Verbrennung frei von Endothelzellen waren, wurden in der Nähe der transplantierten IPE-Zellen lokalisiert. Tabelle 2 fasst die Klassifikation der Laserverbrennungen basierend auf der Größe der CNV-Gebiete in verschiedenen Versuchsgruppen zusammen. Sowohl EGFP-exprimierende IPEals auch nicht transduzierte IPE-Transplantate beeinflussten die laserinduzierte CNV-Bildung nicht.

Folglich verhinderte die subretinale Transplantation von PEDF-exprimierenden IPE-Zellen die Neoangiogenese in einem Modell der laserinduzierten Neovaskularisation.

**Tabelle 2: Klassifikation der Laserverbrennungen basierend auf der Größe der CNV-Gebiete.**

| Versuchsgruppe | Laserkontrollen | | Nahe bei PEDF-exprimierenden IPE-Zellen | | Nahe bei EGFP-exprimierende n IPE-Zellen | | Nahe bei nicht transduzierte n IPE-Zellen | |
|---|---|---|---|---|---|---|---|---|
| Beurteilte Laserverbrennungen | n=31 | (%) | n=18 | (%) | n=6 | (%) | n=5 | (%) |
| I. Verbrennungen vollständig vaskularisiert | 28 | 90,3 | 0 | 0 | 6 | 100 | 5 | 100 |
| II. Verbrennungen völlig frei von neuen Gefäßen | 0 | 0 | 6 | 33,3 | 0 | 0 | 0 | 0 |
| III. Verbrennungen mit vaskularisierten und nicht vaskularisierten Gebieten | 3 | 9,7 | 12 | 66,6 | 0 | 0 | 0 | 0 |

### 14. Wirkungen der HC-Ad-Vektor-mediierten PEDF-Expression in transplantierten IPE-Zellen auf den Photorezeptorerhalt in einem Modell der retinalen Degeneration

Zwei Monate nach Transplantation der IPE-Zellen in 20 Tage alte RCS-Ratten (Schraermeyer, U., Kociok, N. & Heimann, K. (1999) Invest Ophthalmol Vis Sci 40, 1545-1556) wurden die Tiere getötet und 5 µm dicke Paraffinschnitte durch das halbe Auge hergestellt. Nach H&E-Färbung wurden die Erhaltungseffekte durch Bestimmung der Zahl der Reihen und die Größe der Gebiete mit erhaltenen Photorezeptornuklei in allen Schnitten bestimmt. Die Rhodopsinexpression wurde mit einem Rhodopsinantikörper (5 µg/ml, Leinco Technol.) und einem Peroxidase-markierten 2. Antikörper nachgewiesen. Die Größe der Gebiete mit erhaltenen Rhodopsin-enthaltenden äußeren Segmenten wurde mittels computergestützter Morphometrie bestimmt. Nur wenige Photorezeptornuklei waren in der Nähe von IPE-Zellen anwesend, die nur mit EGFP-exprimierenden HC-AdFK7-Vektor transduziert waren. Dennoch waren einige Reihen von Photorezeptornuklei angrenzend an mit HC-Ad.PEDF-Vektor transduzierten IPE-Transplantaten erhalten. In diesen Gebieten konnte PEDF, das von HC-Ad.PEDF-transduzierten IPE-Transplantaten exprimiert wurde, durch Immunfluoreszenzmikroskopie gefunden werden. In diesen Schnitten war die Zahl der Photorezeptorreihen signifikant höher (4,4 + 0,68, P<0,05) als in den HC-AdFK7-transduzierten (2,18 + 0,29) und nicht-transduzierten IPE-Transplantaten (2,2 + 0,55). Darüber hinaus hatten in den Schnitten mit PEDF-exprimierenden Zellen (n=9) die Gebiete mit erhaltenen Photorezeptornuklei (mehr als 5 Reihen) eine Länge von 2,6 + 1,0 mm. Erhaltenes Rhodopsin war in den äußeren Stäbchensegmenten der überlebenden Photorezeptoren in der Nähe der PEDF-exprimierenden Zellen vorhanden. In den selben Schnitten wurde eine Rhodopsin positive Fläche von 53 947 + 24 656 µm² (n=5) gemessen. In Schnitten mit HC-AdFK7-transduzierten oder in nicht-transduzierten IPE-Transplantaten war keine Färbung mit Rhodopsin nachweisbar. Daher schützte die subretinale Transplantation von PEDF-exprimierenden IPE-Zellen die Photorezeptordegeneration in einem Modell der retinalen Degeneration.

### 15. Wirkungen von HC-Ad-Vektor-mediierter PEDF-Expression in RPE-Zellen in einem Modell der laserinduzierten choroidalen Noevaskularisation

5 x 10⁶ infektiöse Partikel von HC-Ad.PEDF und HC-AdFK7 in einem Endvolumen von 0,5 µl wurden in den subretinalen Raum 4 bis 5 Monate alter Long Evans Ratten injiziert. Danach wurde eine Laserphotocoagulation (100 µm Bestrahlungsfläche, 0,1 sec. Dauer, 150 mW) unter Verwendung einer Blau-Grün-Einstellung eines kohärenten Novus 2000 Argonlasers (Coherent Inc., USA) nahe an der Stelle der Injektion (3 Verbrennungen/Auge) durchgeführt. 10 Tage nach der Transplantation wurde die choroidale Neovaskularisation in RPE-choroidalen "Flatmounts" durch Markierung der Endothelzellen mit einem Ratte-Anti-Maus-CD31 (PECAM-1) monoklonalen Antikörper (1 µg/ml; Becton Dickinson) bestimmt. Die Größe der Gebiete, die mit Endothelzellen bedeckt waren, wurde durch computergestützte Morphometrie bestimmt. Die Verbrennungen wurden, wie oben erläutert, in die Klassen Typ I, Typ II und Typ III eingeteilt (siehe Tabelle 2).

In solchen Läsionen waren immer Gebiete vorhanden, die frei von Endothelzellen waren. Die Expression von PEDF durch die Transplantate wurde unter Verwendung spezifischer Antikörpern untersucht. In den Kontrollen (nur Laserverbrennungen) waren nur 10 % der Laserverbrennungen ohne Neovaskularisation, wohingegen in den anderen 90 % der Gebiete dieser Narben vollständig mit Endothelzellen gefüllt waren, die durch Färbung mittels eines CD31-Antikörpers nachgewiesen wurden. Alle Narben (100 %) waren vollständig frei von neugebildeten Endothelzellen, wenn PEDF-exprimierende RPE-Zellen sich in der Umgebung der Läsionen im Abstand von 100 µm oder weniger befanden. In diesem Modell wurde eine choroidale Neovaskularisation durch immunologische Methoden in der selben Laserläsion nachgewiesen. Folglich verhinderte die Injektion des PEDF-exprimierenden HC-Ad-Vektors in den subretinalen Raum die Neoangiogenese in einem Modell der Laser-induzierten Neovaskularisation.

### 16. Wirkungen von HC-Ad-Vektor-mediierter PEDF-Expression in RPE-Zellen in einem Modell der sauerstoffinduzierten retinalen Neovaskularisation

In diesen Experimenten wurden Wistar-Ratten verwendet. Die Tiere der normoxischen Gruppe wurden während des Experiments bei Raumluft gehalten. Ratten der hyperoxischen Gruppe wurden vom 7. (P7) bis zum 12. Lebenstag (P12) 75 %igem Sauerstoff ausgesetzt, dann in Raumluft übertragen und sofort subretinal 5 x 10⁶ infektiöse Partikel des HC-Ad.PEDF- oder des HC-AdFK7-Vektors in einem Endvolumen von 0,5 µl injiziert. Bei P22 wurden die Tiere anästhesiert und mit 50 mg/ml Fluoreszeinisothiozyanat-Dextran (Sigma, Deisenhofen, Deutschland) perfundiert. Die Neovaskularisation wurde an retinalen RPE-choroidalen "Flatmounts" unter Verwendung eines Fluoreszenzmikroskops untersucht. Um die Neovaskularisation zu quantifizieren, wurde die Länge der neu gebildeten gewundenen Blutgefäße mit einem Durchmesser von mehr als 25 µm auf der inneren Oberfläche der peripheren Retina durch computergestützte Morphometrie (Openlab software, Impro Vision Inc., Lexington, USA) untersucht. Die Messungen der Gefäßlänge wurden in Gebieten von 800 000 µm²/Auge im Abstand von bis zu 200 µm von den transfizierten RPE-Zellen oder den entsprechenden peripheren Gebiet der hyperoxischen Gruppe durchgeführt. Darüber hinaus wurde zur Bestätigung der epiretinalen Lokalisation der oberflächlichen pathologischen Gefäße 10 µm dicke serielle Gefrierschnitte durch das halbe Auge hergestellt.

Bei P22 wurden auf den "Flatmounts" von einer Hyperoxie ausgesetzten, nichttransplantierten Tieren große Gebiete mit erweiterten radialen Gefäßen, Mikroaneurysmen und Hämorrhagien, die typisch für retinale Neovaskularisation sind, beobachtet. Diese waren in den Retinae der Normoxie-Kontrollen nicht vorhanden. Typische Zeichen von peripherer retinaler Neovaskularisation, die durch vaskuläre Knäuel gekennzeichnet, nämlich erweiterte Gefäße mit abnormalem gewundenem Verlauf auf der inneren Oberfläche der peripheren Retina waren offensichtlich. Die epiretinale Position der neu gebildeten pathologischen Gefäße wurde in Gefrierschnitten beobachtet. Transfizierte RPE-Zellen wurden in den peripheren Gebieten der "Flatmounts" lokalisiert. Transfizierte RPE-Zellen wurden durch EGFP-Expression identifiziert. HC-Ad.PEDF-transduzierte RPE-Zellen verhinderten die Bildung von pathologisch gewundenen Gefäßen in den Gebieten der Transfektion. Immunfluoreszenzmikroskopie zeigte die Expression von PEDF aus genetisch modifizierten Zellen. Transduzierte RPE-Zellen wurden in der Peripherie lokalisiert, wo typische pathologische Gefäße nach Hyperoxie-Exposition in diesem Tiermodell für gewöhnlich gebildet werden. In einem Abstand von 200 µm oder mehr von den transfizierten RPE-Zellen waren erweiterte, gewundene, neu gebildete Gefäße auf der inneren Oberfläche der Retina mit einem Durchmesser von mehr als 25 µm regelmäßig vorhanden. Ähnlich wie in den normoxischen Kontrollen waren in der Nähe der PEDF-exprimierenden RPE-Zellen keine pathologischen, epiretinalen Gefäße vorhanden.

Folglich kann die Sauerstoff-induzierte retinale Neovaskularisation lokal durch HC-Ad-Vektor vermittelte PEDF-Expression in RPE-Zellen gehemmtt werden.

### 17. Statistik

Alle Daten stellen Mittelwerte + SEM von mindestens 3 Experimenten mit 3 bis 6 Einzelbestimmungen dar. Für Mehrfachvergleiche wurde eine "einseitige Analyse der Varianz" (one-way analysis of varinace; ANOVA) mit anschließender post hoc-Analyse (Duncan-Test) verwendet.

## Patentansprüche

1. Pigmentepithelzelle des Auges, **dadurch gekennzeichnet, dass** die Zelle Vektor-DNA eines adenoviralen Vektors mit großer DNA-Kapazität enthält und langfristig stabil mindestens ein eingeführtes Gen exprimiert.

2. Pigmentepithelzelle des Auges nach Anspruch 1, wobei es sich um eine retinale Pigmentepithelzelle oder um eine Irispigmentepithelzelle handelt.

3. Pigmentepithelzelle des Auges nach Anspruch 1 oder 2, wobei die Vektor-DNA mindestens eine therapeutische Nukleinsäure, insbesondere ein therapeutisches Gen, bevorzugt für einen neurotrophen Faktor wie GDNF, PEDF, NGF, BDNF, CNTF, bFGF oder Neurotrophin 3,4-5, einen antiangiogenetischen Faktor wie einen löslichen VEGF-Rezeptor-1 (sflt-1), einen dominant-negativen VEGF-Rezeptor-2 (KDR) oder PEDF, einen antioxidativen Faktor wie Superoxiddismutase, Katalase oder verschiedene Peroxydasen, einen lysosomalen Faktor wie alpha-Mannosidase, beta-galactosidase, N-acetyl-beta-glucosaminidase, N-acetyl-betagalactosaminidase sowie Lipase, oder einen gefäßerweiternden Faktor wie NO-Synthase, enthält.

4. Pigmentepithelzelle des Auges nach mindestens einem der Ansprüche 1-3,
wobei die Vektor-DNA einen konstitutiv aktiven, regulierbaren und/oder einen gewebespezifischen Promotor und/oder ein regulierbares Expressionssystem enthält.

5. Pigmentepithelzelle des Auges nach mindestens einem der Ansprüche 1-4, wobei die Zelle mindestens ein therapeutisches Protein und/oder eine therapeutische RNA produziert.

6. Pigmentepithelzelle des Auges nach mindestens einem der Ansprüche 1-5, wobei die Zelle in einem festen Zellverband ist und/oder in Gegenwart eines "feeder layer" und/oder in Serum-freiem Medium kultiviert worden ist.

7. Pigmentepithelzelle des Auges nach einem der Ansprüche 1-6 in Form eines festen Zellverbands.

8. Kultivierungssystem enthaltend mindestens eine Pigmentepithelzelle des Auges nach einem der Ansprüche 1-7 und einen "feeder layer".

9. In-vitro-Verfahren zur Herstellung einer Pigmentepithelzelle des Auges gemäß mindestens einem der Ansprüche 1-7, **dadurch gekennzeichnet, dass** die Zelle mit Hilfe eines adenoviralen Vektors mit großer DNA-Kapazität genetisch modifiziert wird.

10. Verfahren zur Herstellung einer Pigmentepithelzelle des Auges nach mindestens einem der Ansprüche 1-7, **dadurch gekennzeichnet, dass** die Zelle in Serum-freiem Medium und/oder in Gegenwart eines "feeder layer" kultiviert wird.

11. Verwendung einer Pigmentepithelzelle des Auges gemäß mindestens einem der Ansprüche 1-7 zur Herstellung eines Arzneimittels zur Therapie einer Augenerkrankung, insbesondere AMD, einem Glaukom, diabethischer Retinopathie oder einer genetisch bedingten Erkrankung des Pigmentepithels.

12. Verwendung einer Pigmentepithelzelle des Auges gemäß mindestens einem der Ansprüche 1-7 zur Herstellung eines Arzneimittels zur Therapie einer Nervenerkrankung, insbesondere einer Erkrankung des Nervensystems, bevorzugt des ZNS vor allem von Morbus Parkinson.

13. Verwendung einer Pigmentepithelzelle des Auges nach mindestens einem der Ansprüche 11-12, **dadurch gekennzeichnet, dass** die Pigmentepithelzelle eine autologe Pigmentepithelzelle ist.

14. Arzneimittel oder Diagnostikum enthaltend eine Pigmentepithelzelle des Auges gemäß mindestens einem der Ansprüche 1-7 sowie weitere Hilfs-und/oder Zusatzstoffe.

15. Pigmentepithelzelle des Auges gemäß mindestens einem der Ansprüche 1-7 zur Verwendung bei der Therapie einer Augenerkrankung, insbesondere AMD, einem Glaukom, diabethischer Retinopathie oder einer genetisch bedingten Erkrankung des Pigmentepithels.

16. Pigmentepithelzelle des Auges gemäß mindestens einem der Ansprüche 1-7 zur Verwendung bei der Therapie einer Nervenerkrankung, insbesondere einer Erkrankung des Nervensystems, bevorzugt des ZNS vor allem von Morbus Parkinson.

17. Pigmentepithelzelle des Auges zur Verwendung nach mindestens einem der Ansprüche 15-16, **dadurch gekennzeichnet, dass** die Pigmentepithelzelle eine autologe Pigmentepithelzelle ist.

## Claims

1. Pigment epithelial cell of the eye, **characterized in that** the cell contains vector DNA of an adenoviral vector with large DNA capacity and stably expresses in the long term at least one introduced gene.

2. Pigment epithelial cell of the eye according to claim 1, wherein the pigment epithelial cell is a retinal pigment epithelial cell or an iris pigment epithelial cell.

3. Pigment epithelial cell of the eye according to claim 1 or 2, wherein the vector DNA contains at least one therapeutic nucleic acid, in particular a therapeutic gene, preferably for a neurotrophic factor such as GDNF, PEDF, NGF, BDNF, CNTF, bFGF or neurotrophin 3,4-5, an anti-angiogenetic factor such as a soluble VEGF receptor-1 (sflt-1), a dominant-negative VEGF receptor-2 (KDR) or PEDF, an anti-oxidative factor such as superoxide dismutase, catalase or various peroxydases, a lysosomal factor such as alpha-mannosidase, beta-galactosidase, N-acetyl-beta-glucosaminidase, N-acetyl-beta-galactosaminidase, and lipase, or a vasodilating factor such as NO synthase.

4. Pigment epithelial cell of the eye according to at least one of the claims 1-3, wherein the vector DNA contains a constitutively active, regulatable and/or a tissue-specific promoter and/or a regulatable expression system.

5. Pigment epithelial cell of the eye according to at least one of the claims 1-4, wherein the cell produces at least one therapeutic protein and/or one therapeutic RNA.

6. Pigment epithelial cell of the eye according to at least one of the claims 1-5, wherein the cell is in a fixed assemblage of cells and/or was cultivated in presence of a feeder layer and/or in serum-free medium.

7. Pigment epithelial cell of the eye according to any one of the claims 1-6 in form of a fixed assemblage of cells.

8. Cultivation system containing at least one pigment epithelial cell of the eye according to any one of the claims 1-7 and a feeder layer.

9. *In-vitro* method for producing a pigment epithelial cell of the eye according to at least one of the claims 1-7, **characterized in that** the cell is genetically modified with the help of an adenoviral vector with large DNA capacity.

10. Method for producing a pigment epithelial cell of the eye according to at least one of the claims 1-7, **characterized in that** the cell is cultivated in serum-free medium and/or in presence of a feeder layer.

11. Use of a pigment epithelial cell of the eye according to at least one of the claims 1-7 for producing a medicament for the therapy of an eye disease, in particular of AMD, a glaucoma, diabetic retinopathy or a genetic disease of the pigment epithelium.

12. Use of a pigment epithelial cell of the eye according to at least one of the claims 1-7 for producing a medicament for the therapy of a nervous disease, in particular a disease of the nervous system, preferably of the CNS, especially of Parkinson's disease.

13. Use of a pigment epithelial cell of the eye according to at least one of the claims 11-12, **characterized in that** the pigment epithelial cell is an autologous pigment epithelial cell.

14. Medicament or diagnostic aid containing a pigment epithelial cell of the eye according to at least one of the claims 1-7 and further excipients and/or additives.

15. Pigment epithelial cell of the eye according to at least one of the claims 1-7 for use in the therapy of an eye disease, in particular of AMD, a glaucoma, diabetic retinopathy or a genetic disease of the pigment epithelium.

16. Pigment epithelial cell of the eye according to at least one of the claims 1-7 for use in the therapy of a nervous disease, in particular a disease of the nervous system, preferably of the CNS, especially of Parkinson's disease.

17. Pigment epithelial cell of the eye for use according to at least one of the claims 15-16, **characterized in that** the pigment epithelial cell is an autologous pigment epithelial cell.

## Revendications

1. Cellule épithéliale pigmentaire de l'oeil, **caractérisée en ce que** la cellule comprend de l'ADN vecteur d'un vecteur adénoviral ayant une grande capacité ADN et exprime de manière stable à long terme au moins un gène introduit.

2. Cellule épithéliale pigmentaire de l'oeil selon la revendication 1, qui est une cellule épithéliale pigmentaire rétinienne ou une cellule épithéliale pigmentaire de l'iris.

3. Cellule épithéliale pigmentaire de l'oeil selon la revendication 1 ou 2, dans laquelle l'ADN vecteur comprend au moins un acide nucléique thérapeutique, en particulier un gène thérapeutique, de préférence pour un facteur neurotrophique comme GDNF, PEDF, NGF, BDNF, CNTF, bFGF ou la neurotrophine 3,4-5, un facteur anti-angiogénique comme un récepteur soluble de type 1 du VEGF (sflt-1), un récepteur de type 2 du VEGF à dominante négative (KDR) ou PEDF, un facteur anti-oxydant comme la superoxyde dismutase, la catalase ou diverses peroxydases, un facteur lysosomal comme l'alpha-mannosidase, la bêta-galactosidase, la N-acétyl-bêta-glucosaminidase, la N-acétyl-bêta-galactosaminidase, ainsi que la lipase, ou un facteur vasodilatateur comme la NO-synthase.

4. Cellule épithéliale pigmentaire de l'oeil selon au moins une des revendications 1 à 3, dans laquelle l'ADN vecteur comprend un promoteur constitutivement actif, régulable et/ou un promoteur spécifique à un tissu et/ou un système d'expression régulable.

5. Cellule épithéliale pigmentaire de l'oeil selon au moins une des revendications 1 à 4, dans laquelle la cellule produit au moins une protéine thérapeutique et/ou un ARN thérapeutique.

6. Cellule épithéliale pigmentaire de l'oeil selon au moins une des revendications 1 à 5, dans laquelle la cellule est dans un amas cellulaire solide et/ou a été cultivée en présence d'une « couche nourricière » et/ou dans un milieu exempt de sérum.

7. Cellule épithéliale pigmentaire de l'oeil selon une des revendications 1 à 6 sous la forme d'un amas cellulaire solide.

8. Système de culture comprenant au moins une cellule épithéliale pigmentaire de l'oeil selon une des revendications 1 à 7 et une « couche nourricière ».

9. Procédé *in vitro* de préparation d'une cellule épithéliale pigmentaire de l'oeil selon au moins une des revendications 1 à 7, **caractérisé en ce que** la cellule est modifiée génétiquement à l'aide d'un vecteur adénoviral ayant une grande capacité ADN.

10. Procédé de préparation d'une cellule épithéliale pigmentaire de l'oeil selon au moins une des revendications 1 à 7, **caractérisé en ce que** la cellule est cultivée dans un milieu exempt de sérum et/ou en présence d'une « couche nourricière ».

11. Utilisation d'une cellule épithéliale pigmentaire de l'oeil selon au moins une des revendications 1 à 7 pour la préparation d'un médicament destiné à la thérapie d'une affection oculaire, en particulier de la DMLA, d'un glaucome, de la rétinopathie diabétique ou d'une affection de l'épithélium pigmentaire d'origine génétique.

12. Utilisation d'une cellule épithéliale pigmentaire de l'oeil selon au moins une des revendications 1 à 7 pour la préparation d'un médicament destiné à la thérapie d'une maladie nerveuse, en particulier d'une maladie du système nerveux, de préférence du SNC surtout de la maladie de Parkinson.

13. Utilisation d'une cellule épithéliale pigmentaire de l'oeil selon au moins une des revendications 11 et 12, **caractérisée en ce que** la cellule épithéliale pigmentaire est une cellule épithéliale pigmentaire autologue.

14. Médicament ou diagnostic comprenant une cellule épithéliale pigmentaire de l'oeil selon au moins une des revendications 1 à 7 ainsi que d'autres auxiliaires et/ou additifs.

15. Cellule épithéliale pigmentaire de l'oeil selon au moins une des revendications 1 à 7 pour l'utilisation dans la thérapie d'une affection oculaire, en particulier de la DMLA, d'un glaucome, de la rétinopathie diabétique ou d'une affection de l'épithélium pigmentaire d'origine génétique.

16. Cellule épithéliale pigmentaire de l'oeil selon au moins une des revendications 1 à 7 pour l'utilisation dans la thérapie d'une maladie nerveuse, en particulier d'une maladie du système nerveux, de préférence du SNC surtout de la maladie de Parkinson.

17. Cellule épithéliale pigmentaire de l'oeil pour l'utilisation selon au moins une des revendications 15 et 16, **caractérisée en ce que** la cellule épithéliale pigmentaire est une cellule épithéliale pigmentaire autologue.
